# EUROPEAN PATENT APPLICATION

(11) **EP 1 845 083 A2**
(43) Date of publication of application: **17.10.2007**
(21) Application number: 07468004.2
(22) Date of filing: 11.04.2007
(51) Int. Cl.: C07C 311/48, A61K 31/18, A61P 31/04

(54) **New arylsulfonohydrazide inhibitors of enzymes MurC and MurD**

(30) Priority: 13.04.2006 SI 200600094
(71) Applicant: UNIVERZA V LJUBLJANI, FAKULTETA ZA FARMACIJO, 1000 Ljubljana (SI)
(72) Inventor: Obreza, Ales, 1270 Litija (SI); Frlan, Rok, 4223 Poljane (SI); Vobovnik, Nina, 2364 Ribnica na Pohorju (SI); Kovac, Andreja, 9226 Moravske Toplice (SI); Blanot, Didier, 91940 Les Ulis (FR); Pecar, Slavko, 1235 Radomlje (SI); Gobec, Stanislav, 1000 Ljubljana (SI)

(57) **Abstract**

This invention belongs to the field of pharmaceutical chemistry and relates to new arylsulfonohydrazides as inhibitors of UDP-*N*-acetylmuramyl:L-alanine ligaze (MurC) and UDP-N-acetylmuramyl-L-alanine:D-glutamate ligaze (MurD), to procedures for their preparation and pharmaceutical preparations containing the same. The enzymes MurC and MurD are the key enzymes involved in the synthesis of bacterial peptidoglycan, so arylsulfonohydrazide inhibitors possess antibacterial activity. Compounds of general formula I 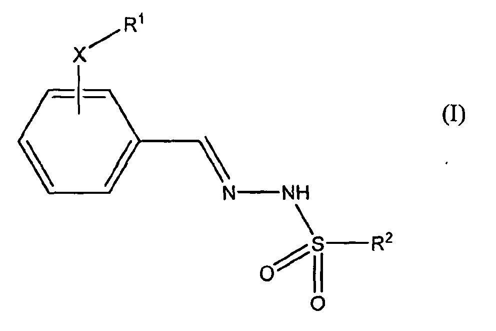 and the pharmaceutically acceptable salts are described. The appropriate substituents are clearly presented in the body of the text and in claims.

## Description

This invention belongs to the field of pharmaceutical chemistry and relates to new arylsulfonohydrazides as inhibitors of UDP-*N*-acetylmuramyl:L-alanine ligaze (MurC) and UDP-*N*-acetylmuramyl-L-alanine:D-glutamate ligaze (MurD), to procedures for their preparation and pharmaceutical preparations containing the same. The enzymes MurC and MurD are the key enzymes involved in the synthesis of bacterial peptidoglycan, so arylsulfonohydrazide inhibitors possess antibacterial activity.

### Technical problem

The widespread and growing presence of pathogenic bacterial strains with resistance to antibiotics is a serious danger for public health (Ritter, T.K.; Wong, C.-H. Angew. Chem. Int. Ed. 2001, 40, 3508-3533; Davies, J. Nature 1996, 383, 219-220; Levy, S.B. Sci.Am. 1998, 278, 46-53). Some life threating bacterial strains (like *Enterococcus faecalis, Mycobacterium tuberculosis, Pseudomonas aeuginosa*) have developed resistance to all antibiotics in clinical use. This situation has generated an urgent need to discover novel antibacterial agents directed towards previously unused targets

### The state of technique

Peptidoglycan is an essential bacterial cell-wall polymer unique to prokaryotic cells and therefore optimal for the selective targeting of microbial vital pathways. When properly constructed (linear glycan chains interlinked by short peptides), peptidoglycan provides the rigidity, flexibility and strength that are necessary for the bacterial cells to grow and divide, while withstanding high internal osmotic pressure. The early steps of peptidoglycan biosynthesis take place in the cytoplasm and represent suitable targets for drug discovery.

Amino acid ligases MurC, MurD, MurE and MurF catalize the addition of L-alanine (L-Ala), D-glutamic acid (D-Glu), *meso*-diaminopimelic acid or L-lysine (L-Lys), and dipeptide D-alanyl-D-alanine, respectively, to the growing UDP-*N*-acetylmuramoyl-pentapeptide (van Heijenoort, J. Nat. Prod. Rep. 2001, 18, 503-519). All Mur ligases are promising targets for development of new antibacterial agents as they are present in eubacteria but not in mammals. Inhibitors of Mur ligases are thus expected to have selective and broad-spectral antibacterial activity (van Heijenoort, J. Nat. Prod. Rep. 2001, 18, 503-519; van Heijenoort, J. Biosynthesis of the peptidoglycan unit. (1994) In Ghuysen, J.M. and Hakenbeck, R. (eds), Bacterial Cell Wall. Elsevier Science B.V., Amsterdam, pp. 39-54).

MurC (UDP-*N*-acetylmuramyl:L-alanine ligaze) is cytoplasmatic bacterial enzyme which catalyzes the addition of L-alanine to the nucleotide precursor UDP-*N*-acetylmuramic acid (UDP-Mur*N*Ac) according to the reaction:

***UDP-MurNAc + L-Ala + ATP* ↔ *UDP-MurNAc-L-Ala + ADP + Pi***

MurD (UDP-*N*-acetylmuramyl-L-alanine:D-glutamate ligaze) is cytoplasmatic bacterial enzyme which catalyzes the addition of D-glutamic acid to the nucleotide precursor UDP-*N*-acetylmuramoyl-L-alanine (UDP-MurNAc-L-Ala) according to the reaction:

***UDP-MurNAc-L-Ala* + *D-Glu* + *ATP*** ↔ ***UDP-MurNAc-L-Ala-D-Glu* + *ADP* + *Pi***

The mechanism of amino acid addition for both enzymes is similar to other ATP-dependent ligases (like glutamine synthetase, glutathion synthetase, D-Ala-D-Ala ligase). The enzyme first phosphorylates the free carboxylic acid of the nucleotide substrate with the γ-phosphate group of ATP so that the acyl-phosphate intermediate is formed. The activated carboxylic acid is then attacked by the amino group of the incoming L-alanine (MurC) or D-glutamic acid (MurD). The reaction proceeds via a high-energy tetrahedral intermediate and ends by the release of free phosphate and the peptide bond containing product. This mechanism was confirmed by biochemical studies (Vaganay, S.; Tanner, M.E.; van Heijenoort, J.; Blanot, D. Microbial Drug Resistance 1996, 2, 51-54; Bouhss, A.; Dementin, S.; van Heijenoort, J.; Parquet, C.; Blanot, D. FEBS Letters 1999, 453, 15-19) and crystalographic analysis of enzyme-substrate/product complexes (Bertrand, J.A.; Auger, G.; Martin, L.; Fanchon, E.; Blanot, D.; Le Beller, D.; van Heijenoort, J; Dideberg, O. J. Mol. Biol. 1999, 289, 579-590). Additional verification for the mechanisms described above are phosphinate transition-state analogue inhibitors of MurC (Reck, F.; Marmor, S.; Fisher, S.; Wuonola, M.A. Bioorg. Med. Chem. Lett. 2001, 11, 1451-1454) and MurD (Tanner, M.E.; Vaganay, S.; van Heijenoort, J.; Blanot, D. J. Org. Chem. 1996, 61, 1756-1760; Gegnas, L.D.; Waddell, S.T.; Chabin, R.M.; Reddy, S.; Wong, K.K. Bioorg. Med. Chem. Lett. 1998, 8, 1643-1648; Gegnas L. patent WO 98/43651; 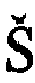 trancar, K.; Blanot, D.; Gobec, S. Bioorg. Med. Chem. Lett. 2006, 16, 343-348).
These are the first known inhibitors of MurC and MurD. Although some of them are very potent enzyme inhibitors (for example 1 nM ≤ IC₅₀ < 680 nM for MurD from *E*. *coli),* they have some unfavourable properties like high hydrophilicity, presence of more than 10 H-bond acceptors, high molecular mass (more than 500). According to "Lipinski rule of five" they are not appropriate for new drug discovery (Lipinski, C.A.; Lombardo, F.; Dominy, B.W.; Feeney, P.J. Advanced Drug Delivery Reviews 2001, 46, 3-26). So far, only few small-molecule inhibitors of MurC and MurD were synthesized that correspond to the "Lipinski rule of five". Benzylidene rhodanines are inhibitors of MurC that also possess antibacterial activity against some Gram positive bacteria (Sim, M.M.; Ng, S.B.; Buss, A.D.; Crasta, S.C.; Goh, K.L.; Lee, S.K. Bioorg. Med. Chem. Lett. 2002, 12, 697-699). Some derivatives of 2-phenyl-5,6-dihydro-2H-thieno[3,2-c]pyrazol-3-ol inhibited MurC and also MurB and MurD (Li, Z.; Francisco, G.D.; Hu, W.; Labthavikul, P.; Petersen, P.J.; Severin, A.; Singh, G.; Yang, Y.; Rasmussen, B.A.; Lin, Y.; Skotnicki, J.S.; Mansour, T.S. Bioorg. Med. Chem. Lett. 2003, 13, 2591-2594). Benzofuran acyl-sulfonamide compound was published as new a type of MurC inhibitor (Ehmann, D.E.; Demeritt, J.E.; Hull, K.G.; Fisher, S.L. Biochim. Biophys. Acta 2004, 1698, 167-174).

Small-molecule inhibitors of MurD are some *N*-acylated D-glutamic acid derivatives (IC₅₀ >= 44 µM for MurD from *S*. *pneumoniae*) (Victor, F.; Tebbe, M.J.; Birch, G.B.; Smith, M.C.; Letourneau, D.L.; Wu, C.E. Abstracts of the 39th Interscience Conference on Antimicrobial Agents and Chemotherapy, San Francisco, 1999, page 330, abstract 1276), simple analogues of the transition-state of MurD (IC₅₀ >= 83 µM for MurD from *S. Pneumoniae*) (Snyder, N.J., Tebbe, M.J., Victor, F. et al. Abstracts of the 39th Interscience Conference on Antimicrobial Agents and Chemotherapy, San Francisco, 1999, page 330, abstract 1275) and macrocyclic inhibitors (IC₅₀ >= 700 nM for MurD from *E. Coli)* (Horton, J.R.; Bostock, J.M.; Chopra, I.; Hesse, L.; Phillips, S.E.V.; Adams, D.J.; Johnson, A.P.; Fishwick, C.W.G. Bioorg. Med. Chem. Lett. 2003, 13, 1557-1560). For these inhibitors of MurD no antibacterial activity has been demonstrated so far.

### Description of solution of the technical problem including examples

The invention relates to novel arylsulfonohydrazide derivatives and analogs thereof of the general formula (I) 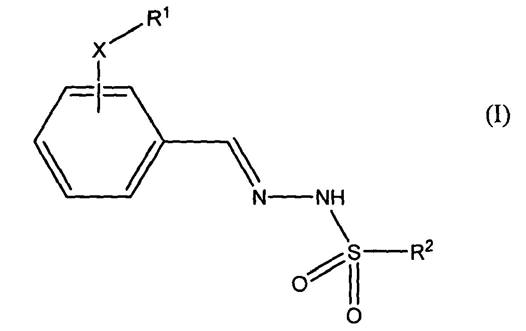 wherein
R¹ is a residue, which may be nonsubstituted, mono, di, polysubstituted phenyl, benzyl or phenethyl or polycyclic aromatic ring or combination of five and sixmembered ring with one or more heteroatoms. Some examples are presented in the formula below 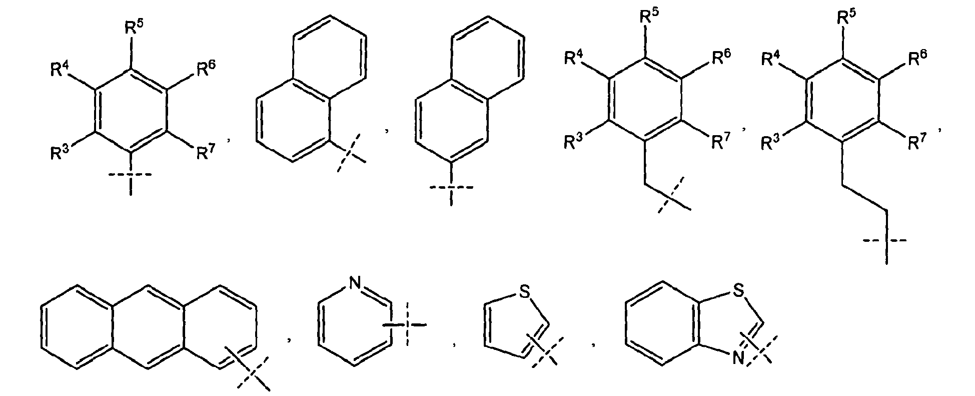 wherein R³, R⁴, R⁵, R⁶ in R⁷ = H, alkyl (C₁-C₆), OH, NH₂, SH, NO₂, SO₂NH₂, SOCH₃, SO₂CH₃, CF₃, F, Cl, Br, I, NHCOCH₃; R³, R⁴, R⁵, R⁶ and R⁶ may form additional carbocyclic or heterocyclic ring with 1 , 2 or more heteroatoms,
R² represents nonsubstituted, mono, di, polysubstituted phenyl, benzyl or polycyclic aromatic ring of the formula 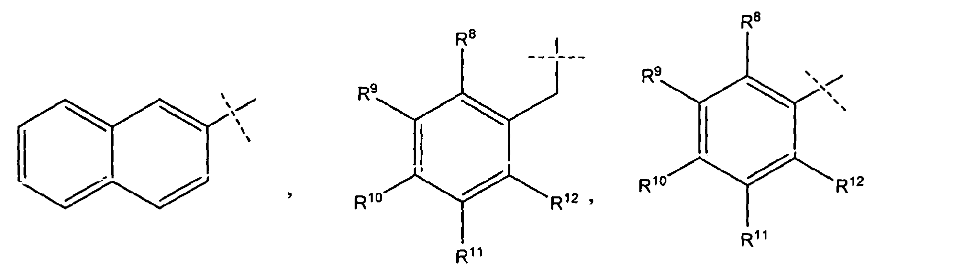 wherein R⁸, R⁹, R¹⁰, R¹¹ in R¹² = H, alkyl (C₁-C₆), OH, NH₂, SH, NO₂, SO₂NH₂, SOCH₃, SO₂CH₃, CF₃, F, Cl, Br, I, NHCOCH₃;
X represents a residue of the formula: -OCH₂-, -NHCO-, -CONH, -OSO₂-, -COO-, -OOC-,-O-, -S-, -NH-

The invention also relates to their pharmaceutically acceptable salts with an inhibitory activity on enzymes UDP-*N*-acetylmuramate-L-alanine ligase (MurC) and UDP-*N-*acetylmuramoyl-L-alanine:D-glutamate ligase (MurD) and to pharmaceutical compositions containing them. The invention also relates to a process for the preparation of arylsulfonohydrazide derivatives and analogs thereof of the general formula (I).

Arylsulfonohydrazide derivatives and analogs thereof of the general formula (I), when X = -OCH₂- are prepared as follows:
Suitably substituted hydroxybenzaldehyde of formula (II) 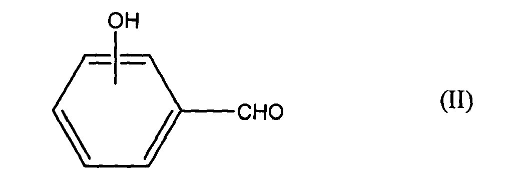 is converted in the presence of KF and suitable base with substituted arylalkylchloride of formula (III) 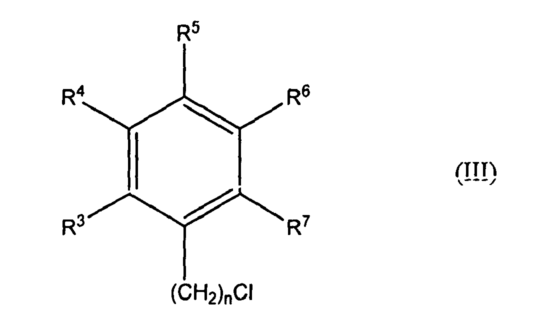
wherein n=1-3, R³, R⁴, R⁵, R⁶ and R⁷ have the same meanings as in the formula (I), or with substituted arylalkylchloride of formula (IV) 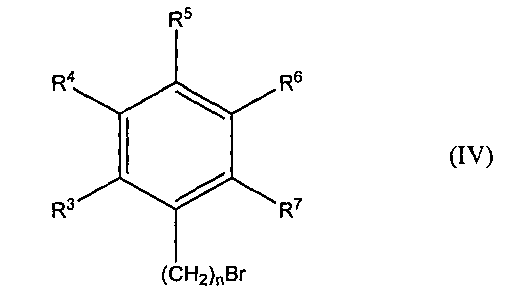 wherein n=1-3; R³, R⁴, R⁵, R⁶ and R⁷ have the same meanings as in the formula (I), to the compound (V) 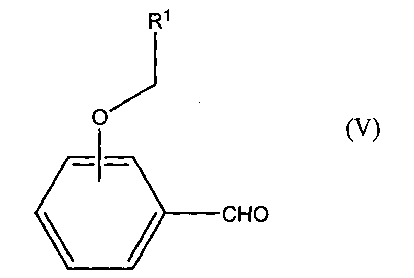 wherein R¹ has the same meaning as in the formula (I), which reacts with aryl-, arylalkyl-, or heteroaryl- sulfonohydrazide of the formula (VI) 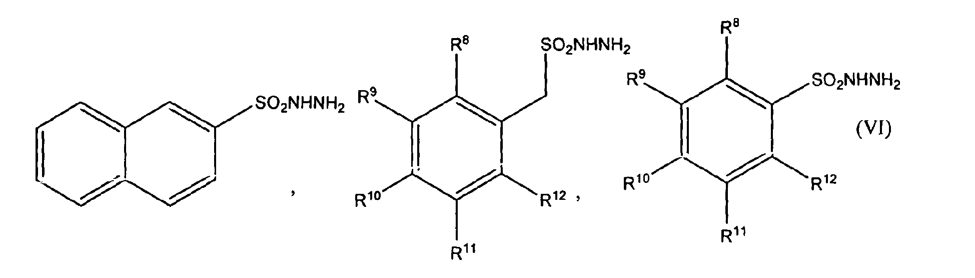 wherein R⁸, R⁹, R¹⁰, R¹¹ in R¹² have the same meanings as in the formula (I), to the compound of the formula (VII), 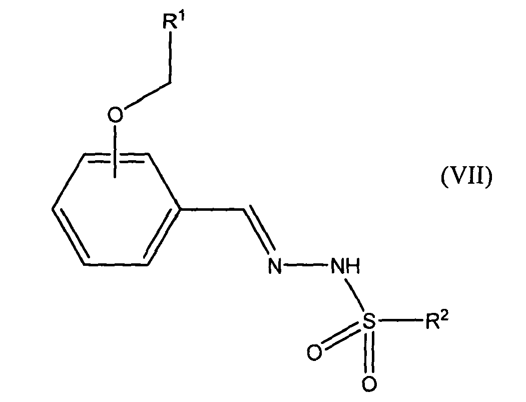 wherein R¹ in R² have the same meanings as in the formula (I).

Arylsulfonohydrazide derivatives and analogs thereof of the general formula (I), wherein X = -OSO₂- are prepared as follows:
Suitably substituted hydroxybenzaldehyde of the formula (II) 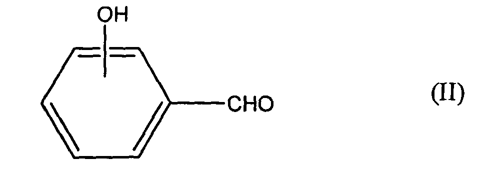 is converted with substituted arylsulfonylchloride or arylalkylsulfonylchloride of formula (VIII) 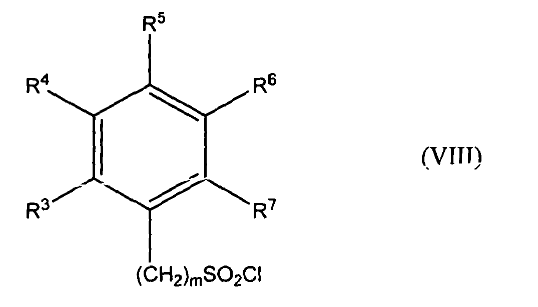
wherein m=0-2; R³, R⁴, R⁵, R⁶ and R⁷ have the same meanings as in the formula (I), to the compound of the formula (IX) 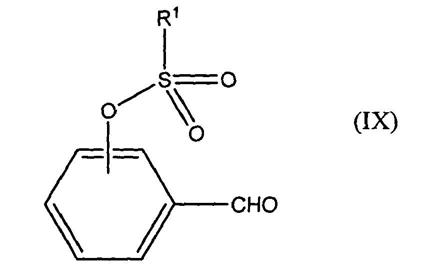 wherein R¹ has the same meaning as in the formula (I),
which is transformed with aryl-, arylalkyl-, or heteroaryl- sulfonohydrazide of the formula (VI) 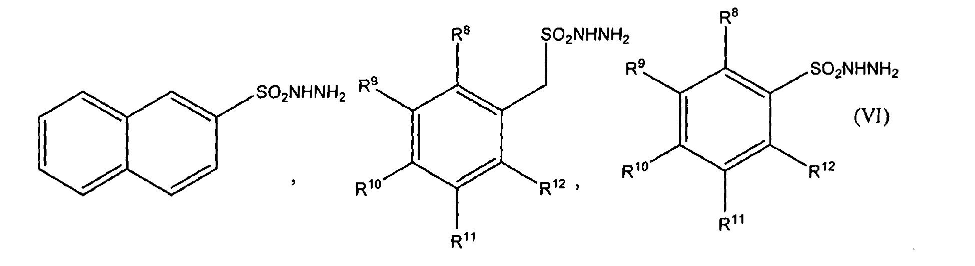 wherein R⁸, R⁹, R¹⁰, R¹¹ and R¹² have the same meanings as in the formula (I), to the compound of the formula (X), 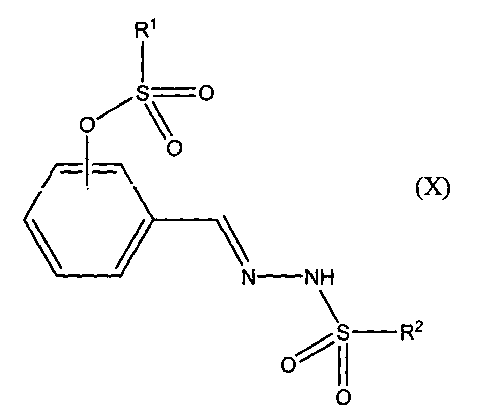 wherein R¹ and R² have the same meanings as in the formula (I).

Arylsulfonohydrazide derivatives and analogs thereof of the general formula (I), wherein X = -NHCO- are prepared as follows:
Suitably substituted nitrobenzaldehyde of the formula (XI) 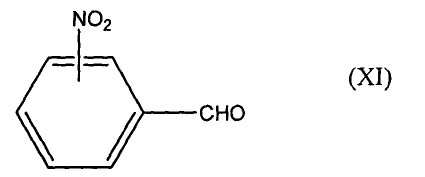 is protected with ethyleneglycol, to the compound of the formula (XII), 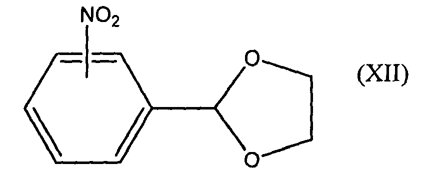 which by subjecting to reduction by catalytic hydrogenation, using Pd as a catalyst, and by the use of substituted benzoylchloride or arylalkanoylchloride of formula (XIII) 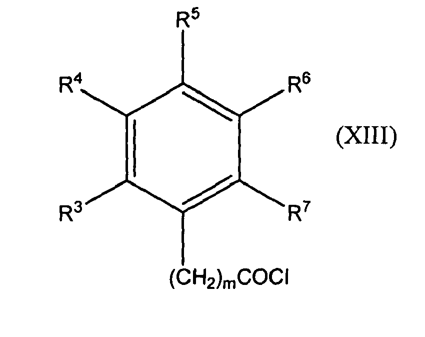
wherein m=0-2; R³, R⁴, R⁵, R⁶ and R⁷ have the same meanings as in the formula (I), is converted to the compound of the formula (XIV), 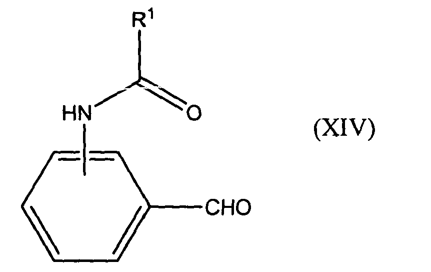 wherein R¹ has the same meaning as in the formula (I),
which is transformed with aryl-, arylalkyl-, or heteroaryl- sulfonohydrazide of the formula (VI) 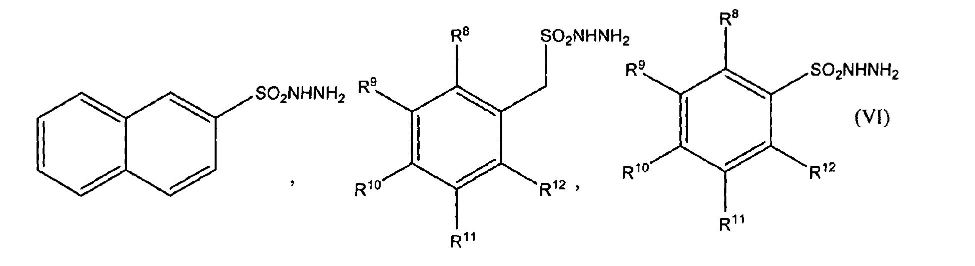 wherein R⁸, R⁹, R¹⁰, R¹¹ and R¹² have the same meanings as in the formula (I), to the compound of the formula (XV), 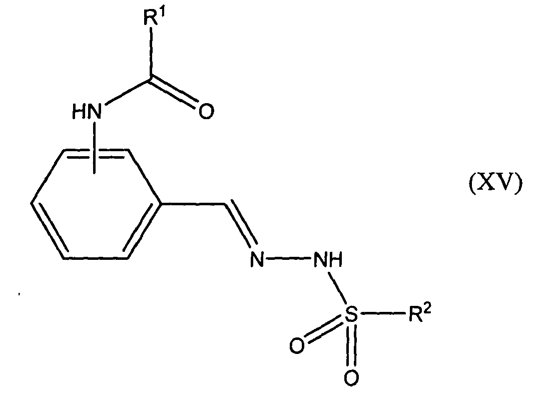 wherein R¹and R² have the same meanings as in the formula (I).

Arylsulfonohydrazide derivatives and analogs thereof of the general formula (I), wherein X = -CONH- are prepared as follows:
Suitably substituted carboxybenzaldehyde of the formula (XVI) 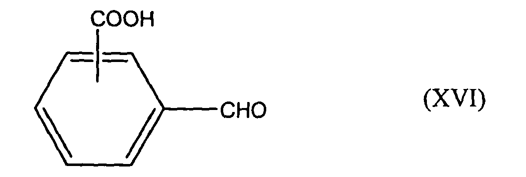 is reacted with aryl-, arylalkyl-, or heteroaryl- sulfonohydrazide of the formula (VI) 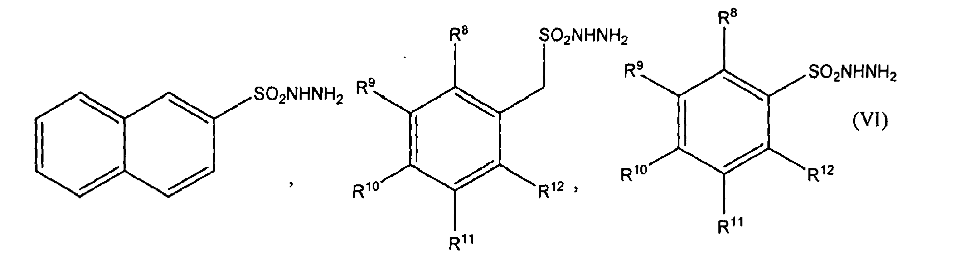
wherein R⁸, R⁹, R¹⁰, R¹¹ and R¹² have the same meanings as in the formula (I), to the compound of the formula (XVII), 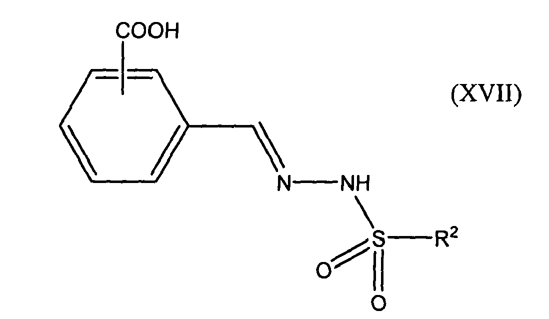 wherein R² has the same meaning as in the formula (I).
Compound (XVII) is converted with aniline, arylamine or heteroarylamine derivative of the formula (XVIII) 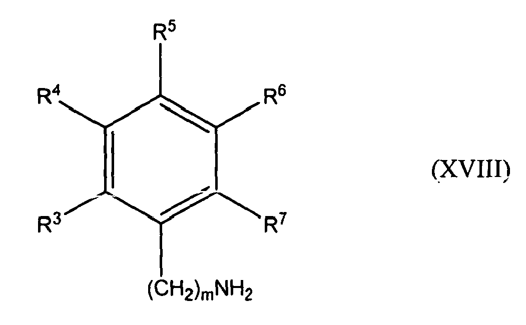 wherein m =0-2; R³, R⁴, R⁵, R⁶ and R⁷ have the same meanings as in the formula (I), in the presence of EDC and HOBT or DCC or DPPA or BOP to the compound of the formula (XIX) 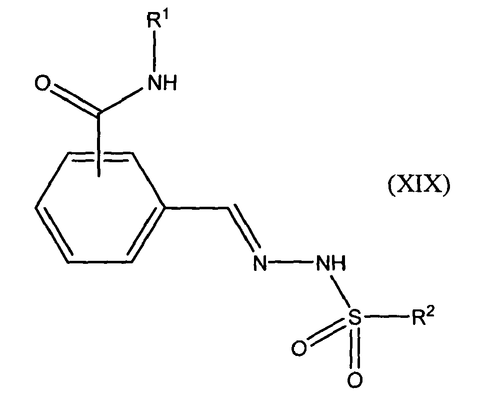 wherein R¹and R² have the same meanings as in the formula (I).

Arylsulfonohydrazide derivatives and analogs thereof of the general formula (I), wherein X = -COO- are prepared as follows:
Suitably substituted carboxybenzaldehyde of the formula (XVI) 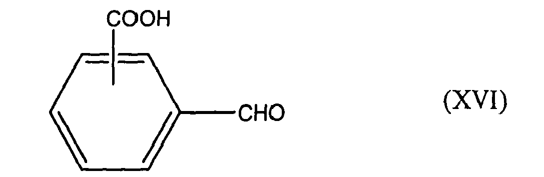 is reacted with aryl-, arylalkyl-, or heteroaryl- sulfonohydrazide of the formula (VI) 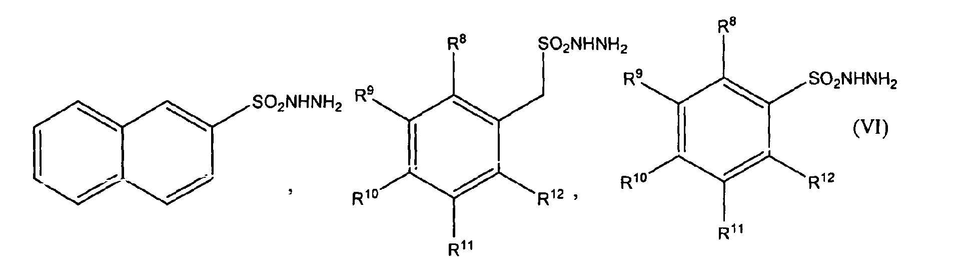
wherein R⁸, R⁹, R¹⁰, R¹¹ and R¹² have the same meanings as in the formula (I), to the compound of the formula (XVII), 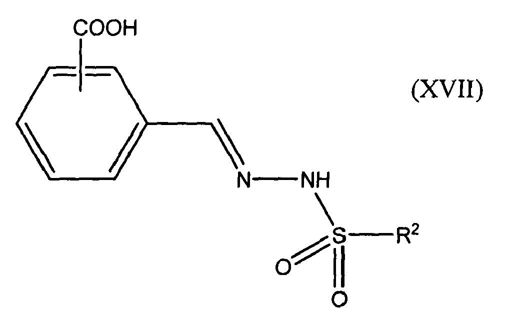 wherein R² has the same meaning as in the formula (I).
Compound (XVII) is converted with suitably substituted phenol or arylalkylalcohol of the formula (XX) 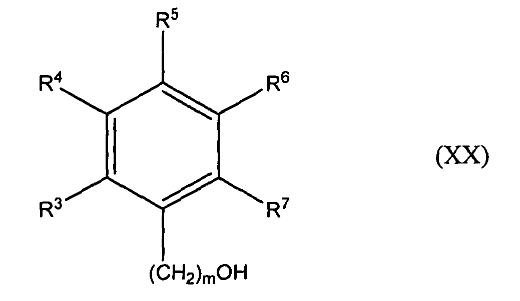 wherein m =0-2; R³, R⁴, R⁵, R⁶ and R⁷ have the same meanings as in the formula (I), in the presence of EDC and HOBT or DCC or DPPA or BOP to the compound of the formula (XXI) 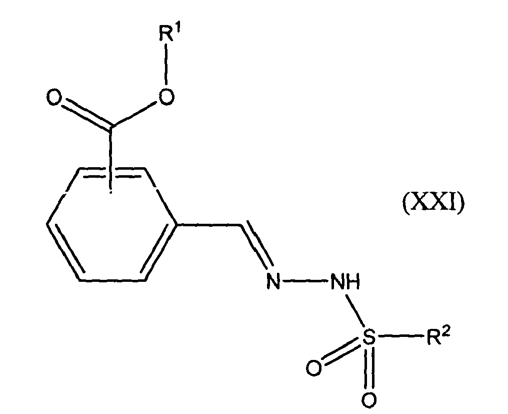 wherein R¹and R² have the same meanings as in the formula (I).
Arylsulfonohydrazide derivatives and analogs thereof of the general formula (I), wherein X = -OOC- are prepared as follows:
Suitably substituted hydroxybenzaldehyde of formula (II) 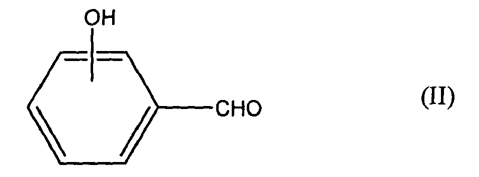 Is transformed in the presence of EDC and HOBT or DCC or DPPA or BOP with substituted benzoic acid, or arylalcanoic acid of the formula (XXII) 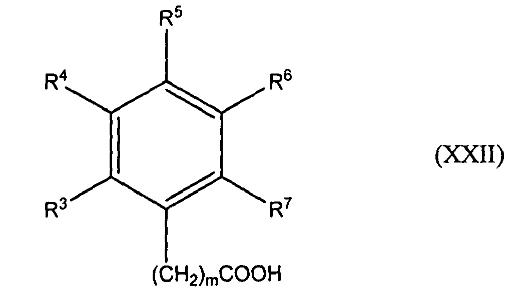 wherein m =0-2; R³, R⁴, R⁵, R⁶ and R⁷ have the same meanings as in the formula (I), or with substituted benzoylchloride or arylalkanoylchloride of formula (XIII) 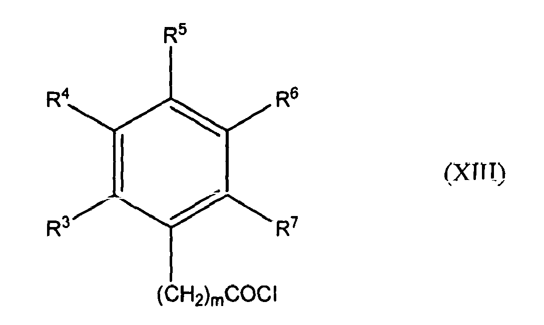 wherein m =0-2; R³, R⁴, R⁵, R⁶ and R⁷ have the same meanings as in the formula (I), to the compound of the formula (XXIII) 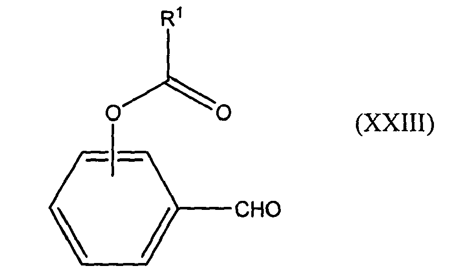 wherein R¹ has the same meaning as in the formula (I),
   which is reacted with aryl-, arylalkyl-, or heteroaryl- sulfonohydrazide of the formula (VI) 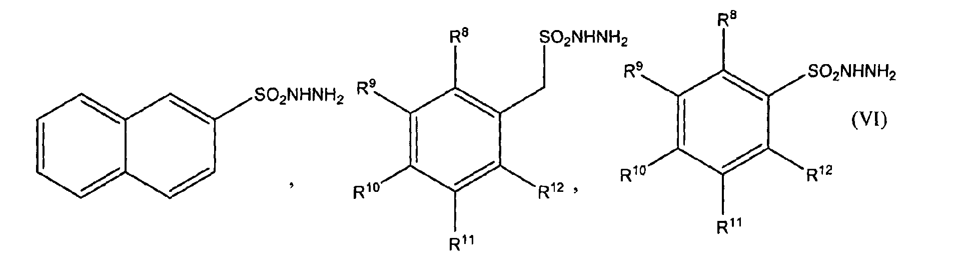 wherein R⁸, R⁹, R¹⁰, R¹¹ and R¹² have the same meanings as in the formula (I), to the compound of the formula (XXIV), 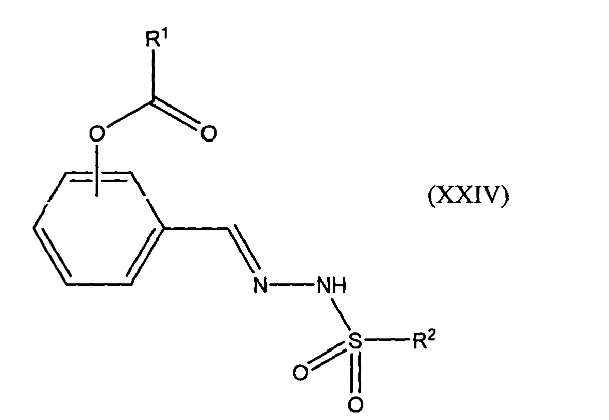 wherein R¹ and R² have the same meanings as in the formula (I).

Arylsulfonohydrazide derivatives and analogs thereof of the general formula (I), wherein X = -O- are prepared as follows:
Suitably substituted aryloxibenzaldehyde or heteroaryloxibenzaldehyde of the formula (XXV), 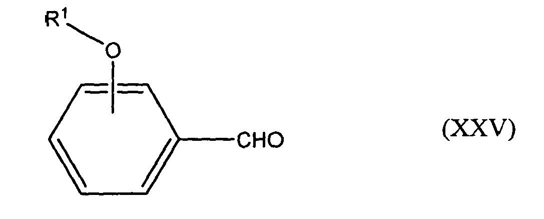
wherein R¹ has the same meaning as in the formula (I), is reacted with aryl-, arylalkyl-, or heteroaryl- sulfonohydrazide of the formula (VI) 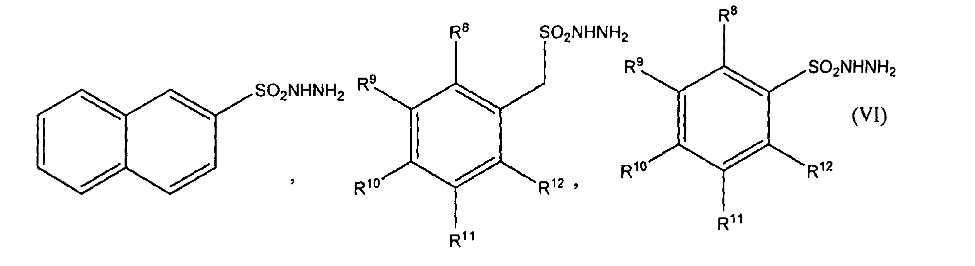 wherein R⁸, R⁹, R¹⁰, R¹¹ and R¹² have the same meanings as in the formula (I), to the compound of the formula (XXVI), 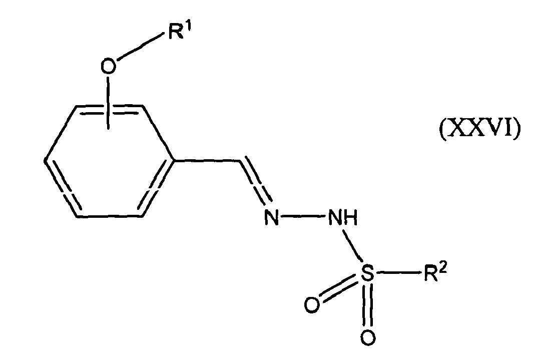 wherein R¹ and R² have the same meanings as in the formula (I).

### Biological data

Inhibitory activity of compounds against MurC (MurD) from *E.coli* [1] was tested for their ability to inhibit the addition of L-Ala (D-Glu) to UDP-Mur*N*ac (UDP-Mur*N*Ac-L-Ala). Detection of orthophosphate generated during the reaction was based on the colorimetric malachite green method as described elsewhere [2] with slight modifications using a mixture (final volume: 50 µl) containing 50 mM Hepes, pH 8.0, 3,25 mM MgCl₂, 120 µM UDP-Mur*N*Ac (80 µM UDP-Mur*N*Ac-L-Ala), 120 µM L-Ala (100 µM D-Glu), 450 µM ATP (400 µM ATP), purified MurC (MurD) from *E.coli* (diluted with 20 mM Hepes, pH 7.2, 1 mM dithiothreitol) and 100 µM of tested compound dissolved in DMSO. Final concentration of DMSO did not exceed 5% (v/v). Compounds that weren't soluble in the enzyme assay mixture were tested in concentrations lower than 100 µM. Reaction mixture was then incubated at 37°C for 15 min and after quenched with 100 µl of Biomol^{®} reagent. Absorbance was measured after 5 min at 650 nm. Residual activity was calculated with respect to a similar assay without inhibitor. IC₅₀ constants were determined by measuring residual activity at seven different inhibitor concentrations and represent the concentration of inhibitors where RA is 50%.
[1] Auger, G.; Martin, L.; Bertrand, J.; Ferrari, P.; Fanchon, E.; Vaganay, S.; Pétillot, Y.; van Heijenoort, J.; Blanot, D.; Dideberg, O. Large-scale preparation, purification, and crystallization of UDP-N-acetylmuramoyl-L-alanine: D-glutamate ligase from Escherichia coli. Prot. Express. Purif. 1998, 13, 23-29.
[2] Lanzetta, P.A.; Alvarez, L.J.; Reinach, P.S., Candia, O. An improved assay for nanomole amounts of inorganic phosphate. Anal. Biochem. 1979, 100, 95.

| | **MurD** | | **MurC** | |
|---|---|---|---|---|
| **Structure** | **RA (%)** | **IC₅₀** | **RA (%)** | **IC₅₀** |
| 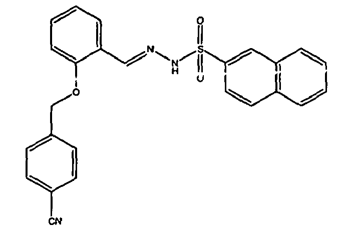 | 27 (100µM) 74 (25µM) | | 36 (100µM) | |
| 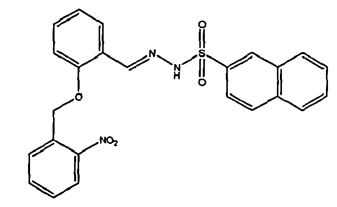 | 32 (100µM) 87 (25µM) | | 30 (100µM) | |
| 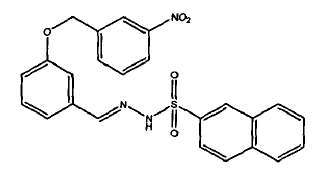 | 61(50µM) | | 58 (50µM) | |
| 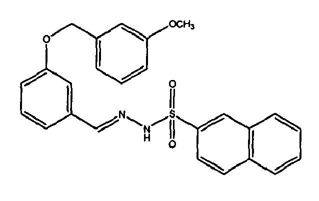 | 69 (50µM) | | 52 (50µM) | |
| 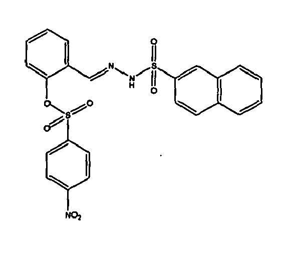 | 25 (100µM) 78 (25µM) | | 75 (100µM) | |
| 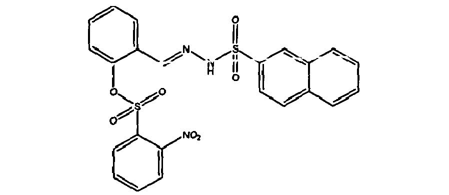 | 71 (µM) | | 55 (50µM) | |
| 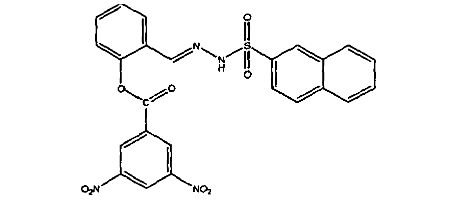 | 33 (100µM) 83 (25µM) | | 45 (100µM) | |
| 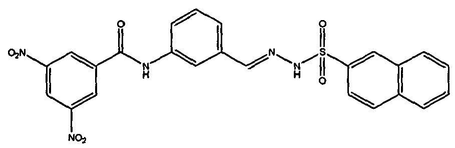 | 4(100µM) 88 (10µM) | | 51(100µ) | |
| 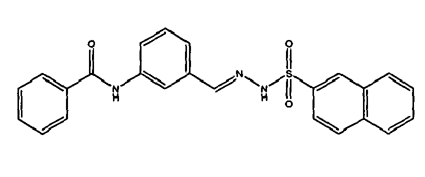 | 56(100µM) | | 97 (100µM) | |
| 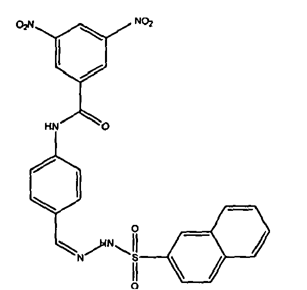 | 78 (100µM) | | 72(100µM) | |
| 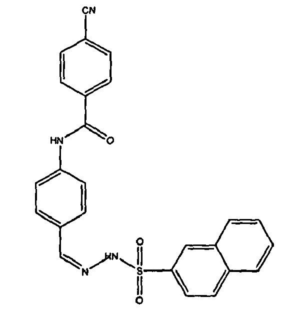 | 78 (50µM) | | 91 (50µM) | |
| 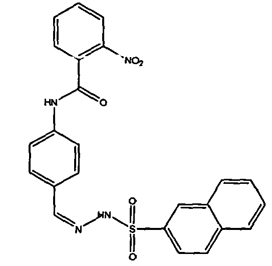 | 1 (100µM) 80 (10µM) | | 27 (50µ) | **51** (µM) |
| 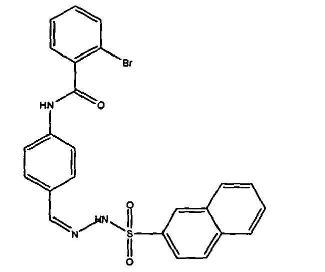 | 13 (100µM) 84 (10µM) | | 53 (50µ) | |
| 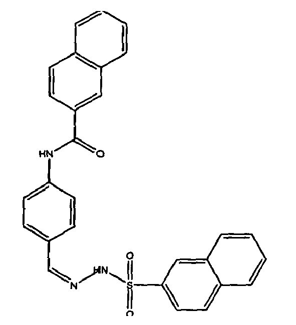 | 19 (10µM) 84 (2µM) | | 27 (100µM) | **31** (µM) |
| 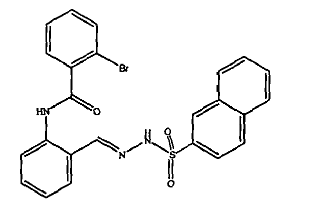 | 4 (100µM) 53 (10µM) 81 (5µM) | | 23 (100µM) | **35** (µM) |
| 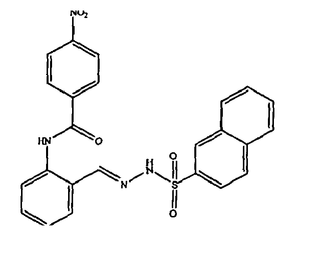 | 4 (100µM) 66 (10µM) | | 19 (100µM) | **27** (µM) |
| 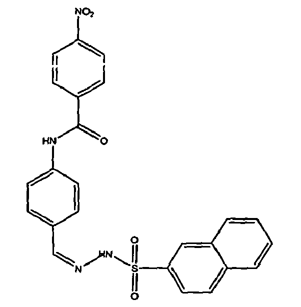 | 81 (50µM) | | 71 (50µM) | |
| 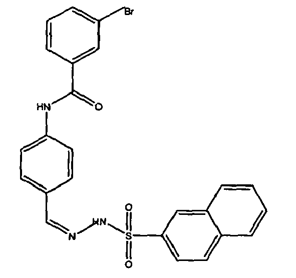 | 41 (10µM) 77 (5µM) | | 9 (100µM) 21 (50µ) | 30 (µM) |
| 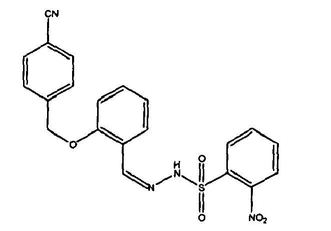 | 86 (100µM) | | 95 (100µM) | |
| 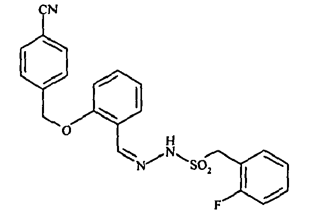 | 39 (100µM) | | 59 (100µM) | |
| 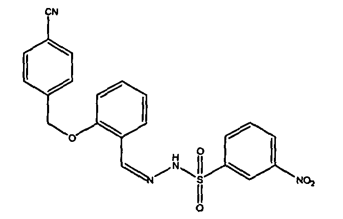 | 86 (50µM) | | | |
| 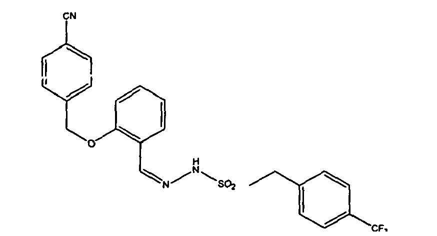 | 57 (50µM) | | | |
| 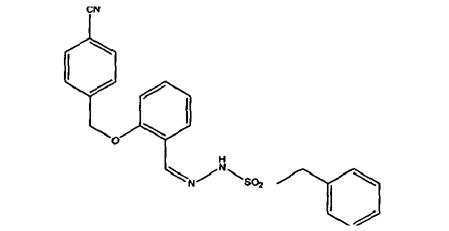 | 58 (100µM) | | 61 (100µM) | |
| 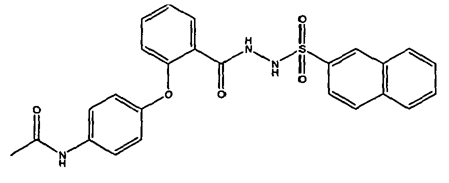 | 93 (100µM) | | 100 (100µM) | |
| 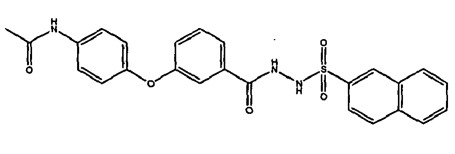 | 94 (100µM) | | 96 (100µM) | |

The invention is illustrated but in no way limited by the following examples:

### EXAMPLE 1: 4-(1,3-dioxolan-2-yl)-aniline

2-(4-nitrophenyl)-1,3-dioxolane (5.76 g (38.1 mmol)) was dissolved in 100 mL THF and hydrogenated for 2 hours. The catalyst was filtered off, and the solution of the product immediatelly used in the next reaction step.

### EXAMPLE 2: N-(4-Formylphenyl)-3,5-dinitrobenzamide

(355 mg (2.57 mmol)) K₂CO₃ and 3,5-dinitrobenzoylchloride (652 mg (2.83 mmol)) were added to the 25 mL of the solution of 4-(1,3-dioxolan-2-yl)-aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 80.2%
**Molecular formula:** C₁₄H₉N₃O₅
**M**=315.238 g/mol
**¹H NMR (300 MHz, DMSO-d₆):** δ 7.97 (d, 2H, J=6.0 Hz, Ar-H), 8.05 (d, 2H, J=7.0 Hz, Ar-H), 9.02 (t, 1H, J=3.4 Hz, Ar-H), 9.19 (d, 2H, J=2.0 Hz, Ar-H), 9.96 (s, 1H, CHO), 11.20 (s, 1H, NH-CO) ppm.
**MS (EI): m/z (%)**: 315 (M⁺, 73), 195 (100).
**IR (KBr):** ν 3340, 3069, 1664, 1598, 1528, 1333, 1271, 1167, 920, 832, 729, 653 cm⁻¹.
**Elemental analysis: Calculated for C₁₄H₉N₃O₅×1/3 H₂O (M=315,238 g/mol):** 52.34 %C; 3.03 %H; 13.08 %N. Measured: 52.62 %C; 3.06 %H; 12.83 %N.
**Melting point**: 258-264°C

### EXAMPLE 3: N-(4-{[2-(2-Naphthylsulfonyl)hydrazono]methyl}phenyl)-3,5-dinitrobenzamide

*N*-(4-Formylphenyl)-3,5-dinitrobenzamide (200 mg (0.634 mmol)) was dissolved in 30 mL absolute alcohol and K₂CO₃ (89 mg (0.634 mmol)) and 2-naphthalenesulfonohydrazinium chloride (163 mg (0.634 mmol)) were added. The reaction mixture was stirred overnight, the solvent was evaporated in vacuo and the residue dissolved in 30 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo.
**Yield:** 71.9%
**Molecular formula:** C₂₄H₁₇N₅O₇S
**M**=519.487 g/mol
**¹H NMR (300 MHz, DMSO-d₆):** δ 7.61 (d, 2H, J=8.7 Hz, Ar-H), 7.70 (m, 2H, Ar-H), 7.83 (d, 2H, J=8.7 Hz, Ar-H), 7.91 (dd, 1H, J₁=7.8 Hz, J₂=1.8 Hz, Ar-H), 7.93 (s, 1H, CH=N-NH), 8.03 (d, 1H, J=8.1 Hz, Ar-H), 8.15 (d, 1H, J=8.8 Hz, Ar-H), 8.23 (d, 1H, J=8.1 Hz, Ar-H), 8.58 (s, 1H, Ar-H), 9.00 (t, 1H, J=2.1 Hz, Ar-H), 9,15 (d, 2H, J=2,1 Hz, Ar-H), 10,95 (s, 1H,
NH-CO), 11,55 (s, 1H, =N-NH-SO₂) ppm.
**MS (FAB): m/z (%):** 520 (MH⁺, 64), 154 (100).
**IR (KBr):** ν 3329, 1674, 1531, 1411, 1341, 1164, 832, 717 cm⁻¹.
**Elemental analysis: Calculated for C₂₄H₁₇N₅O₇S×1/3 H₂O (M=519,487 g/mol):** 54.85 %C; 3.39 %H; 13.33 %N. **Measured:** 54.85 %C; 3.46 %H; 13.02 %N.
**Melting point:** 220-222°C

### EXAMPLE 4: N-(4-Formylphenyl)benzamide

(474 mg (3.43 mmol)) K₂CO₃ and benzoylchloride (530 mg (3.77 mmol)) were added to the 33 mL of the solution of 4-(1,3-dioxolan-2-yl)aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 78.1%
**Molecular formula:** C₁₄H₁₁NO₂
**M**=225.243 g/mol
**¹H NMR (300 MHz, DMSO-d₆):** δ 7.54-7.66 (m, 3H, Ar-H), 7,92 (d, 2H, J=8.7 Hz, Ar-H), 7.98 (d, 2H, J=7.6 Hz, Ar-H), 8.04 (d, 2H, J=8.7 Hz, Ar-H). 9.92 (s, 1H, CHO), 10.64 (s, i H,
NH-CO) ppm.
**MS (EI): m/z (%):** 225 (M⁺, 27), 105 (100).
**IR (KBr):** ν 3336, 2737, 1696, 1659, 1592, 1524, 1415, 1323, 1168, 826, 718 cm⁻¹.
**HRMS: Calculated for: C₁₄H₁₁NO₂:** 225.078979 **Measured:** 225.079580
**Melting point:** 121-123°C

### EXAMPLE 5: N-(4-{[2-(2-Naphthylsulfonyl)hydrazono]methyl}phenyl)benzamide

*N*-(4-Formylphenyl)benzamide (200 mg (0.888 mmol)) was dissolved in 30 mL absolute alcohol and K₂CO₃ (123 mg (0.888 mmol)) and 2-naphthalenesulfonohydrazinium chloride (230 mg (0.888 mmol)) were added. The reaction mixture was stirred overnight, the solvent was evaporated in vacuo and 20 mL EtOAc were added to the residue. The white precipitate was filtered off, washed with EtOAc and dried on air.
**Yield:** 83.3%
**Molecular formula:** C₂₄H₁₉N₃O₃S
**M** = 429.492 g/mol
**¹H NMR (300 MHz, DMSO-d₆):** δ 7.50-7.62 (m, 5H, Ar-H), 7.70 (m, 2H, Ar-H), 7.82 (d, 2H, J=8.7 Hz, Ar-H), 7.93 (m, 3H, Ar-H), 7.93 (s, 1H, CH=N-NH), 8.03 (d, 1H, J=8.1 Hz, Ar-H), 8.14 (d, 1H, J=8.8 Hz, Ar-H), 8.22 (d, 1H, J=8.1 Hz, Ar-H), 8.58 (s, 1H, Ar-H), 10.40 (s, 1H, NH-CO), 11.57 (s, 1H, =N-NH-SO₂) ppm.
**MS (FAB): m/z (%):** 430 (MH⁺, 86), 55 (100).
**IR (KBr):** ν 3339, 3221, 1652, 1580, 1520, 1410, 1320, 1167, 1049, 957, 830, 748, 665, 549 cm⁻¹.
**Elemental analysis: Calculated for C₂₄H₁₉N₃O₃S×3/5 H₂O (M=429, 492 g/mol):** 62.73 %C; 4.40 %H; 9.14 %N. Measured: 62.71 %C; 4.27 %H; 9.18 %N.
**Melting point:** 197-200°C

### EXAMPLE 6: N-(4-Formylphenyl)-2-nitrobenzamide

(474 mg (3.43 mmol)) K₂CO₃ and 2-nitrobenzoylchloride (700 mg (3.77 mmol)) were added to the 33 mL of the solution of 4-(1,3-dioxolan-2-yl)aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo. Product was further purified with column chromatography (silicagel; CH₂Cl₂:hexane =3:1).
**Yield:** 55.0%
**Molecular formula:** C₁₄H₁₀N₂O₄
**M**=270.240 g/mol
**¹H NMR (300 MHz, DMSO-d₆):** δ 7.80-7.83 (m, 2H, Ar-H), 7.88-7.95 (m, 5H, Ar-H), 8.19 (d, 1H, J=8.2 Hz, Ar-H), 9.93 (s, 1H, CHO), 11.09 (s, 1H, NH-CO) ppm.
**MS (EI): m/z (%):** 270 (M⁺, 85), 150 (100).
**IR (KBr): ν** 3333, 1682, 1596, 1524, 1347, 1255, 1164, 897, 834, 705 cm⁻¹.
**HRMS: Calculated for: C₁₄H₁₀N₂O₄:** 270.064057 **Measured:** 270.064850
**Melting point:** 128-130°C.

### EXAMPLE 7: N-(4-{[2-(2-Naphthylsulfonyl)hydrazono]methyl}phenyl)-2-nitrobenzamide

*N*-(4-Formylphenyl)-2-nitrobenzamide (200 mg (0.740 mmol)) was dissolved in 30 mL absolute alcohol and K₂CO₃ (102 mg (0.740 mmol)) and 2-naphthalenesulfonohydrazinium chloride (192 mg (0.740 mmol)) were added. The reaction mixture was stirred overnight, the solvent was evaporated in vacuo and the residue dissolved in 20 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo. The product was recrystallized from ethanol.
**Yield:** 14.9%
**Molecular formula:** C₂₄H₁₈N₄O₅S
**M**=474.490 g/mol
**¹H NMR (300 MHz, DMSO-d₆):** δ 7.56 (d, 2H, J=8,6 Hz, Ar-H), 7.67-7.78 (m, 6H, Ar-H), 7.85-7.92 (m, 2H, Ar-H), 7.91 (s, 1H, CH=N-NH), 8.03 (d, 1H, J=7.4 Hz, Ar-H), 8.13-8.24 (m, 3H, Ar-H), 8.58 (s, 1H, Ar-H), 10.80 (s, 1H, NH-CO), 11.51 (s, 1H, =N-NH-SO₂) ppm.
**MS (FAB): m/z (%):** 475 (MH⁺, 65), 154 (100).
**IR (KBr):** ν 3334, 3090, 1658, 1524, 1326, 1164, 961, 744, 658 cm⁻¹.
**Melting point:** 218-220°C.

### EXAMPLE 8: 4-Cyano-N-(4-formylphenyl)benzamide

K₂CO₃ (180 mg (1.30 mmol)) and 4-cyanobenzoylchloride (216 mg (1.30 mmol)) were added to the 12.7 mL of the solution of 4-(1,3-dioxolan-2-yl)-aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 74.5%
**Molecular formula:** C₁₅H₁₀N₂O₂
**M**=250.252 g/mol
**¹H NMR (300 MHz, DMSO-d₆):** δ 7.94 (d, 2H, J=8.7 Hz, Ar-H), 8.04 (m, 4H, Ar-H), 8.12 (d, 2H, J=11.7 Hz, Ar-H), 9.93 (s, 1H, CHO), 10.84 (s, 1H, NH-CO) ppm.
**MS (EI): m/z (%):** 250 (M⁺, 37), 130 (100).
**IR (KBr):** ν 3350, 2228, 1677, 1591, 1532, 1323, 1166, 832, 757 cm⁻¹.
**HRMS: Calculated for: C₁₅H₁₀N₂O₂:** 250.074228 **Measured:** 250.075050
**Melting point:** 189-192°C.

### EXAMPLE 9: 4-Cyano-N-(4-{[2-(2-naphthylsulfonyl)hydrazono]methyl}phenyl) benzamide

4-Cyano-N-(4-formylphenyl)benzamide (200 mg (0,799 mmol)) was dissolved in 30 mL absolute alcohol and K₂CO₃ (110 mg (0,799 mmol)) and 2-naphthalenesulfonohydrazinium chloride (206 mg (0,799 mmol)) were added. The reaction mixture was stirred overnight, the solvent was evaporated in vacuo and 20 mL EtOAc were added to the residue. The precipitate was filtered off, washed with EtOAc and dried on air.
**Yield:** 30.0%
**Molecular formula:** C₂₅H₁₈N₄O₃S
**M**=454.502 g/mol
**¹H NMR (300 MHz, DMSO-d₆):** δ 7.57 (d, 2H, J=8,7 Hz, Ar-H), 7.69 (m, 2H, Ar-H), 7.80 (d, 2H, Ar-H), 7.90 (dd, 1H, J₁=7.1 Hz, J₂=1.8 Hz, Ar-H), 7.91 (s, 1H, CH=N-NH), 8.00 - 8.03 (m, 3H, Ar-H), 8.12 (q, 3H, J=7.4 Hz, Ar-H), 8.23 (d, 1H, J=8.1 Hz, Ar-H), 8.58 (s, 1H, Ar-H), 10.60 (s, 1H, NH-CO), 11.53 (s, 1H, =N-NH-SO₂) ppm.
**MS (FAB): m/z (%):** 455 (MH⁺, 41), 154 (100).
**IR (KBr):** ν 3342, 3097, 2229, 1654, 1608, 1526, 1414, 1324, 1164, 1054, 961, 637 cm⁻¹.
**Elemental analysis: Calculated for C₂₅H₁₈N₄O₃S×2/3 H₂O (M=454,502 g/mol):** 64.36 %C; 4.18 %H; 12.01 %N. Measured: 64.17 %C; 4.06 %H; 11.85 %N.
**Melting point:** 201-204°C.

### EXAMPLE 10: N-(4-Formylphenyl)-4-nitrobenzamide

K₂CO₃ (442 mg (3,20 mmol)) and 4-nitrobenzoylchloride (653 mg (3,52 mmol)) were added to the 31 mL of the solution of 4-(1,3-dioxolan-2-yl)-aniline in THF. The reaction mixture was stirred for 3 hours at room temperature. The solvent was removed under reduced pressure and 30 mL EtOAc were added to the residue. The precipitate was filtered off, washed with EtOAc and dried on air.
**Yield**: 42.5%
**Molecular formula:** C₁₄H₁₀N₂O₄
**M**=270.240 g/mol
**¹H NMR (300 MHz, DMSO-d₆):** δ 7.90-8.12 (m, 4H, Ar-H), 8.23 (d, 2H, J=8.9 Hz, Ar-H), 8.47 (d, 2H, J=8.8 Hz, Ar-H), 9.92 (s, 1H, CHO) ppm.
**MS (EI): m/z (%)**: 270 (M⁺, 36), 150 (100).
**IR (KBr):** ν 3362, 1674, 1590, 1578, 1319, 1172, 830, 710 cm⁻¹.
**HRMS: Measured for: C₁₄H₁₀N₂O₄:** 270.064057 **Measured:** 270.064860
**Melting point:** 213-216°C.

### EXAMPLE 11: N-(4-{[2-(2-Naphthylsulfonyl)hydrazono]methyl}phenyl)-4-nitrobenzamide

*N*-(4-Formylphenyl)-4-nitrobenzamide (200 mg (0,740 mmol)) was dissolved in 30 mL absolute alcohol and K₂CO₃ (102 mg (0.740 mmol)) and 2-naphthalenesulfonohydrazinium chloride (192 mg (0.740 mmol)) were added. The reaction mixture was stirred overnight, the solvent was evaporated in vacuo and the residue dissolved in 20 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo. The product was recrystallized from ethanol.
**Yield**: 24.3%
**Molecular formula:** C₂₄H₁₈N₄O₅S
**M**=474.496 g/mol
**¹H NMR (300 MHz, DMSO-d₆):** δ 7.58 (d, 2H, J=8.7 Hz, Ar-H), 7.69 (m, 2H, Ar-H), 7.81 (d, 2H, J=8.7 Hz, Ar-H), 7.91 (dd, 1H, J₁=8.0 Hz, J₂=1.8 Hz, Ar-H), 7.92 (s, 1H, CH=N-NH), 8.03 (d, 1H, J=8.1 Hz, Ar-H), 8.13-8.24 (m, 4H, Ar-H), 8.36 (d, 2H, J=8.9 Hz, Ar-H), 8.59 (s, 1H, Ar-H), 10.68 (s, 1H, NH-CO), 11.54 (s, 1H, =N-NH-SO₂) ppm.
**MS (FAB): m/z (%):** 475 (MH⁺, 53), 154 (100).
**IR (KBr):** ν 3338, 3080, 1659, 1601, 1525, 1323, 1162, 1055, 829, 689 cm⁻¹.
**Elemental analysis: Calculated for C₂₄H₁₈N₄O₅S (M=474,496 g/mol):** 60.75 %C; 3.82 %H; 11.81 %N. Measured: 60.23 %C; 3.96 %H; 11.46 %N.
**Melting point:** 244-247°C.

### EXAMPLE 12: 3-Bromo-N-(4-formylphenyl)benzamide

K₂CO₃ (442 mg (3,20 mmol)) and 3-bromobenzoylchloride (653 mg (3,52 mmol)) were added to the 31 mL of the solution of 4-(1,3-dioxolan-2-yl)-aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 94.4%
**Molecular formula:** C₁₄H₁₀BrNO₂
**M**=304.139 g/mol
**¹H NMR (300 MHz, DMSO-d₆):** δ 7.53 (t, 1H, J=7.9 Hz, Ar-H), 7.83 (d, 1H, J=9.0 Hz, Ar-H), 7.91-8.04 (m, 5H, Ar-H), 8.17 (s, 1H, Ar-H), 9.93 (s, 1H, CHO), 10.73 (s, 1H, NH-CO) ppm.
**MS (EI): m/z** (%): 303/305 (M⁺,24),183/185 (100).
**IR (KBr):** ν 3357, 1679, 1587, 1533, 1325, 1165, 838, 710 cm⁻¹.
**HRMS: Calculated for: C₁₄H₁₀BrNO₂:** 302,989490 **Measured:** 302,988320
**Melting point:** 163-165°C.

### EXAMPLE 13: 3-Bromo-N-(4-{[2-(2-naphthylsulfonyl)hydrazono]methyl}phenyl) benzamide

3-Bromo-*N*-(4-formylphenyl)benzamide (200 mg (0,657 mmol)) was dissolved in 30 mL of absolute alcohol and K₂CO₃ (91 mg (0,657 mmol)) and 2-naphthalenesulfonohydrazinium chloride (170 mg (0,657 mmol)) were added. The reaction was synthesized according to the procedure for the synthesis of N-(4-{[2-(2-naftilsulfonil)hidrazono]metil}fenil)-4-nitrobenzamid (EXAMPLE 11).
**Yield:** 19.7%
**Molecular formula:** C₂₄H₁₈BrN₃O₃S
**M**=508.388 g/mol
**¹H NMR (300 MHz, DMSO-d₆):** δ 7.38-7.55 (m, 5H, Ar-H), 7.66-7.73 (m, 5H, Ar-H), 7.89 (dd, 1H, J₁=7.5 Hz, J₂=1.8 Hz, Ar-H), 7.89 (s, 1H, CH=N-NH), 8.02 (d, 1H, J=8.1 Hz, Ar-H), 8.13 (d, 1H, J=8.7 Hz, Ar-H), 8.21 (d, 1H, J=8.1 Hz, Ar-H), 8.57 (s, 1H, Ar-H), 10.61 (s, 1H, NH-CO), 11.48 (s, 1H, =N-NH-SO₂) ppm.
**MS (FAB): m/z (%):** 508/510 (MH⁺, 63), 69 (100).
**IR (KBr):** ν 3363, 3160, 1650, 1526, 1332, 1163, 962, 818, 744, 571 cm⁻¹.
**Elemental analysis: Calculated for C₂₄H₁₈BrN₃O₃S (M=508,388 g/mol):** 56.70 %C; 3.57 %H; 8.27 %N. **Measured:** 56.94 %C; 3.69 %H; 8.42 %N.
**Melting point:** 227-230°C.

### EXAMPLE 14: 2-Bromo-N-(4-formylphenyl)benzamide

K₂CO₃ (442 mg (3,20 mmol)) and 2-bromobenzoylchloride (653 mg (3,52 mmol)) were added to the 31 mL of the solution of 4-(1,3-dioxolan-2-yl)-aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 80.0%
**Molecular formula:** C₁₄H₁₀BrNO₂
**M**=304.139 g/mol
**¹H NMR (300 MHz, DMSO-d₆):** δ 7.43-7.55 (m, 3H, Ar-H), 7.61 (dd, 1H, J₁=6.5 Hz, J₂=1.8 Hz, Ar-H), 7.75 (d, 1H, J=9.0 Hz, Ar-H), 7.90-7.97 (m, 3H, Ar-H), 9.93 (s, 1H, CHO), 10.92 (s, 1H, NH-CO) ppm.
**MS (EI): m/z (%):** 303/305 (M⁺, 17), 183/185 (100).
**IR (KBr):** ν 3247, 1689, 1596, 1530, 1330, 1165, 1026, 896, 833, 744 cm⁻¹.
**HRMS: Calculated for: C₁₄H₁₀BrNO₂:** 302,989490 **Measured:** 302,990210
**Melting point:** 118-120°C

### EXAMPLE 15: 2-Bromo-N-(4-{[2-(2-naphthylsulfonyl)hydrazono]methyl}phenyl) benzamide

2-Bromo-*N*-(4-formylphenyl)benzamide (200 mg (0,657 mmol)) was dissolved in 30 mL absolute alcohol and K₂CO₃ (91 mg (0,657 mmol)) and 2-naphthalenesulfonohydrazinium chloride (170 mg (0,657 mmol)) were added. The reaction mixture was stirred overnight, the solvent was evaporated in vacuo and the residue dissolved in 20 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo. The product was recrystallized from ethanol.
**Yield:** 37.9%
**Molecular formula:** C₂₄H₁₈BrN₃O₃S
**M**=508.388 g/mol
**¹H NMR (300 MHz, DMSO-d₆):** δ 7.52 (qu, 3H, J=7.9 Hz, Ar-H), 7.70 (m, 2H, Ar-H), 7.79 (d, 3H, J=8.7 Hz, Ar-H), 7.89-7.95 (m, 2H, Ar-H), 7.91 (s, 1H, CH=N-NH), 8.03 (d, 1H, J=8.1 Hz, Ar-H), 8.12-8.16 (m, 2H, J=8.1 Hz, Ar-H), 8.23 (d, 1H, J=8.1 Hz, Ar-H), 8.58 (s, 1H, Ar-H), 10.45 (s, 1H, NH-CO), 11.50 (s, 1H, =N-NH-SO₂) ppm.
**MS (FAB): m/z (%):** 508/510 (MH⁺, 86), 57 (100).
**IR (KBr):** ν 3207, 1664, 1588, 1521, 1410, 1322, 1165, 1044, 959, 859, 745, 661 cm⁻¹.
**Elemental analysis: Calculated for C₂₄H₁₈BrN₃O₃S (M = 508,388 g/mol):** 56.70 %C; 3.57 %H; 8.27 %N. Measured: 57.04 %C; 3.77 %H; 7.80 %N.
**Melting point:** 197-200°C.

### EXAMPLE 16: N-(4-Formylphenyl)-2-naphthamide

(442 mg (3,20 mmol)) K₂CO₃ and 2-naphthoylchloride (671 mg (3,52 mmol)) were added to the 31 mL of the solution of 4-(1,3-dioxolan-2-yl)-aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo.
**Yield:** 60.3%
**Molecular formula:** C₁₈H₁₃NO₂
**M**=275.301 g/mol
**¹H NMR (300 MHz, DMSO-d₆):** δ 7.66 (m, 2H, Ar-H), 7.95 (d, 2H, J=8.6 Hz, Ar-H), 8.07 (qu, 6H, J=8.6 Hz, Ar-H), 8.61 (d, 1H, J=9.4 Hz, Ar-H), 9.94 (s, 1H, CHO), 10.80 (s, 1H,
NH-CO) ppm.
**MS (EI): m/z (%):** 275 (M⁺, 87), 155 (100).
**IR (KBr):** ν 3371, 1697, 1664, 1560, 1526, 1413, 1322, 1168, 828, 758, 606 cm⁻¹.
**HRMS: Calculated for: C₁₈H₁₃NO₂:** 275.094629 **Measured:** 275.095240
**Melting point:** 135-137°C.

### EXAMPLE 17: N-(4-{[2-(2-Naphthylsulfonyl)hydrazono]methyl}phenyl)-2-naphthamide

*N*-(4-Formylphenyl)-2-naphthamide (200 mg (0,726 mmol)) was dissolved in 30 mL absolute alcohol and K₂CO₃ (100 mg (0,726 mmol)) and 2-naphthalenesulfonohydrazinium chloride (188 mg (0,726 mmol)) were added. The reaction mixture was stirred overnight, the solvent was evaporated in vacuo and the residue dissolved in 20 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo.
**Yield:** 52.1%
**Molecular formula:** C₂₈H₂₁N₃O₃S
**M**=479.551 g/mol
**¹H NMR (300 MHz, DMSO-d₆):** δ 7.55 (d, 2H, J=8.5 Hz, Ar-H), 7.62-7.71 (m, 4H, Ar-H), 7.85-7.93 (m, 3H, Ar-H), 7.93 (s, 1H, CH=N-NH), 8.00-8.15 (m, 6H, Ar-H), 8.22 (d, 1H, J=7.2 Hz, Ar-H), 8.58 (d, 1H, J=9.7 Hz, Ar-H), 10.58 (s, 1H, NH-CO), 11.58 (s, 1H, =N-NH-SO₂) ppm.
**MS (FAB): m/z (%):** 480 (MH⁺, 71), 154 (100).
**IR (KBr):** ν 3361, 3055, 1650, 1520, 1321, 1162, 964, 819, 661 cm⁻¹.
**Melting point:** 224-227°C.

### EXAMPLE 18: 2-(1,3-Dioxolan-2-yl)-aniline

2-(2-Nitrophenyl)-1,3-dioxolane (5.76 g (38.1 mmol)) was dissolved in 100 mL THF and hydrogenated for 2 hours. The catalyst was filtered off, and the solution of the product immediatelly used in the next reaction step.

### EXAMPLE 19: N-(2-Formylphenyl)-3,5-dinitrobenzamide

K₂CO₃ (345 mg (2,56 mmol)) and 3,5-dinitrobenzoylchloride (649 mg (2,82 mmol)) were added to the 25 mL of the solution of 2-(1,3-dioxolan-2-yl)-aniline in THF. The reaction mixture was stirred for 3 hours at room temperature. The solvent was removed under reduced pressure and 30 mL EtOAc were added to the residue. The precipitate was filtered off, washed with EtOAc and dried on air.
**Yield:** 51.6%
**Molecular formula:** C₁₄H₉N₃O₅
**M**=315.238 g/mol
**¹H NMR (300 MHz, DMSO-d₆):** δ 7.48 (t, 1H, J=7.5 Hz, Ar-H), 7.80 (dt, 1H, J₁=8.4 Hz, J₂=1.8 Hz, Ar-H), 7.97 (dd, 1H, J₁=7.1 Hz, J₂=1.5 Hz, Ar-H), 8.10 (d, 1H, J=8.1 Hz, Ar-H), 9.05 (t, 1H, J=2.1 Hz, Ar-H), 9.16 (d, 2H, J=2.1 Hz, Ar-H), 10.12 (s, 1H, CHO), 11.72 (s, 1H,
NH-CO) ppm.
**MS (EI): m/z (%)**: 315 (M⁺, 49), 287 (100).
**IR (KBr):** ν 3086, 1690, 1531, 1343, 1195, 907, 783, 712 cm⁻¹.
**Elemental analysis: Calculated for C₁₄H₉N₃O₅×1/2 H₂O (M=315,238 g/mol):** 51.86 %C; 3.11 %H; 12.96 %N. Measured: 51.93 %C; 2.84 %H; 12.94 %N.
**Melting point:** 258-260°C.

### EXAMPLE 20: N-(2-{[2-(2-Naphthylsulfonyl)hydrazono]methyl}phenyl)-3,5-dinitrobenzamide

*N*-(2-Formylphenyl)-3,5-dinitrobenzamide (200 mg (0,634 mmol)) was dissolved in 30 mL absolute alcohol and K₂CO₃ (88 mg (0,634 mmol)) and 2-naphthalenesulfonohydrazinium chloride (163 mg (0,634 mmol)) were added. The reaction mixture was refluxed for 5 hours, the solvent was evaporated in vacuo and 20 mL EtOAc were added to the residue. The precipitate was filtered off, washed with 10% citric acid and purified water and dried on air.
**Yield:** 83.0%
**Molecular formula:** C₂₄H₁₇N₅O₇S
**M**=519.487 g/mol
**¹H NMR (300 MHz, DMSO-d₆):** δ 7.30 (t, 1H, J=7.5 Hz, Ar-H), 7.47 (t, 1H, J=7,5 Hz, Ar-H), 7.62-7.78 (m, 5H, Ar-H), 7.99 (d, 1H, J=7,7 Hz, Ar-H), 8.08 (t, 2H, J=8.3 Hz, Ar-H), 8.10 (s, 1H, CH=N-NH), 8.33 (s, 1H, Ar-H), 9.05 (dd, 3H, J₁=12.7 Hz, J₂=2.0 Hz, Ar-H), 11.19 (s, 1H, NH-CO), 11.74 (s, 1H, =N-NH-SO₂) ppm.
**MS (FAB): m/z (%):** 520 (MH⁺, 58), 154 (100).
**IR (KBr):** ν 3239, 3099, 1666, 1582, 1532, 1432, 1349, 1162, 961, 724, 662 cm⁻¹.
**Melting point:** 214-217°C.

### EXAMPLE 21: N-(2-Formylphenyl)benzamide

(345 mg (2,56 mmol)) K₂CO₃ and benzoylchloride (396 mg (2,82 mmol)) were added to the 25 mL of the solution of 2-(1,3-dioxolan-2-yl)aniline in THF. The reaction mixture was stirred for 3 hours at room temperature. The solvent was removed under reduced pressure and the residue dissolved in 30 mL EtOAc. Organic phase was washed with 10% citric acid (20 mL), aqueous solution of NaHCO₃ (20 mL), purified water (20 mL) and brine (20 mL). Organic phase was dried with Na₂SO₄ and the solvent evaporated in vacuo. Product was further purified with column chromatography (silicagel; CH₂Cl₂:hexane = 1:1).
**Yield:** 24.8%
**Molecular formula:** C₁₄H₁₁NO₂
**M**=225.243 g/mol
**¹H NMR (300 MHz, DMSO-d₆):** δ 7.39 (t, 1H, J=7.5 Hz, Ar-H), 7.59-7.70 (m, 3H, Ar-H), 7.63 (dt, 1H, J₁=7.9 Hz, J₂=1.6 Hz, Ar-H), 7.99 (dt, 3H, J₁=7.9 Hz, J₂=1.6 Hz, Ar-H), 8.53 (d, 1H, Ar-H), 10.06 (s, 1H, CHO), 11.75 (s, 1H, NH-CO) ppm.
**MS (EI): m/z (%):** 225 (M⁺, 35), 105 (100).
**IR (KBr):** v 1670, 1589, 1536, 1450, 1291, 1158, 758, 694 cm⁻¹.
**Elemental analysis: Calculated for C₁₄H₁₁NO₂×1/5 H₂O (M=315,238 g/mol):** 73.48 %C; 5.02 %H; 6.12 %N. **Measured:** 73.65 %C; 5.16 %H; 6.05 %N.
**Melting point:** 255-258°C.

### EXAMPLE 22: N-(2-{[2-(2-Naphthylsulfonyl)hydrazono]methyl}phenyl)benzamide

*N*-(2-Formylphenyl)benzamide (100 mg (0,444 mmol)) was dissolved in 30 mL absolute alcohol and K₂CO₃ (62 mg (0,444 mmol)) and 2-naphthalenesulfonohydrazinium chloride (115 mg (0,444 mmol)) were added. The reaction mixture was refluxed for 5 hours, the solvent was evaporated in vacuo and the residue dissolved in 20 mL EtOAc. Organic phase was washed with 10% citric acid (15 mL), aqueous solution of NaHCO₃ (15 mL), purified water (15 mL) and brine (15 mL). Organic phase was dried with Na₂SO₄ and the solvent evaporated in vacuo. The product was recrystallized from ethanol.
**Yield:** 49.6%
**Molecular formula:** C₂₄H₁₉N₃O₃S
**M**=429.492 g/mol
**¹H NMR (300 MHz, DMSO-d₆):** δ 7.07 (t, 1H, J=7.5 Hz, Ar-H), 7.15-7.29 (m, 3H, Ar-H), 7.35 (d, 1H, J=7.9 Hz, Ar-H), 7.47-7.67 (m, 7H, Ar-H), 7.90-7.98 (q, 2H, J=9,1 Hz, Ar-H), 7.95 (s, 1H, CH=N-NH), 8.31-8.41 (m, 2H, Ar-H), 10.04 (s, 1H, NH-CO), 10.98 (s, 1H, =N-NH-SO₂) ppm.
**MS (EI): m/z (%):** 429 (M⁺, 47), 105 (100).
**IR (KBr):** ν 3426, 1674, 1608, 1576, 1518, 1441, 1350, 1162, 895, 754, 708, 554cm⁻¹.
**Melting point:** 202-205°C.

### EXAMPLE 23: 2-(3-Nitrobenzyloxy)benzaldehyde

2-Hydroxybenzaldehyde (242 mg (4,17 mmol)) and 3-nitrobenzylchloride (357 mg (2,082 mmol)) were dissolved in THF. Triethylamine (0,35 mL) and KF (242 mg (4,17 mmol)) were added during stirring and the mixture was refluxed for 12 hours under argon. The solvent was removed under reduced pressure, residue treated with ethylacetate (50 mL) and washed with 10% solution of citric acid (2x 10 mL), saturated solution of NaHCO₃ (2x 10 mL), brine (10 mL) and dried over Na₂SO₄. The solvent was evaporated in vacuo and the crude product purified by column cromatography (silicagel; hexane:EtOAc =4:1).
**Yield:** 87.2 %
**Molecular formula:** C₁₄H₁₁NO₄
**M**=257.25 g/mol
**¹H-NMR(300 MHz, DMSO-d₆):**δ 5.45 (s, 2H, Ar-CH₂), 7.09-7.17 (t, J=7.49 Hz, 1H, Ar-H), 7.30-7.37 (d, J=8.39 Hz, 1H, Ar-H), 7.63-7.79 (m, 3H, Ar-H), 7.98-8.04 (d, J=7.71 Hz, 1H, Ar-H), 8.18-8.25 (d, J=8.10 Hz, 1H, Ar-H), 8.40 (s, 1H, Ar-H), 10.45 (s, 1H, Ar-CHO) ppm.
**MS EI: m/z(%):** 257 (M⁺, 10), 136 (100)
**IR (KBr):** 3452, 1679, 1599, 1528, 1461, 1350, 1238, 1025, 802, 730 cm⁻¹.
**Elemental analysis: Calculated for C₁₄H₁₁NO₄:** 65.37 %C; 4.31 %H; 5.44 %N. Measured: 65.15 %C; 4.46 %H; 5.47 %N.
**Melting point:** 120-122°C.

### EXAMPLE 24: N'-(2-(3-Nitrobenzyloxy)benzylidene)naphthalene-2-sulfonohydrazide

2-(3-Nitrobenzyloxy)benzaldehyde (180 mg (0,700 mmol)) and triethylamine (0.1mL) were dissolved in THF (10 mL) and 2-naphthalenesulfonohydrazinium chloride (185 mg (0,717 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2x 10 mL), purified water (2x 10 mL) and brine (2×10 mL). Organic phase was dried with Na₂SO₄ and the solvent evaporated in vacuo. The product was recrystallized from diethylether/EtOAc.
**Yield:** 80.0 %.
**Molecular formula:** C₂₄H₁₉N₃O₅S
**M**=461.50 g/mol
**¹H-NMR(300 MHz, DMSO-d₆):δ** 5.18 (s, 2H, ArCH₂), 6.79-6.86 (d, J=8.26 Hz, 2H, Ar-H), 7.18-7.27 (t, J=7.72 Hz, 1H, Ar-H), 7.45-7.53 (dd, J₁=7.93 Hz, J₂=1.45 Hz, 1H, Ar-H), 7.65-7.81 (m, 3H, Ar-H), 7.81-7.89 (d, J=7.88 Hz, 1H, Ar-H), 7.91-7.98 (dd, J₁=8.71 Hz, J₂=1.79 Hz, 1H, Ar-H), 8.02 (s, 1H, Ar-H), 8.06-8.12 (d, J=7.66 Hz, 1H, Ar-H), 8.14-8.27 (m, 4H, Ar-H), 8.70 (s, 1H, Ar-CH=), 10.18 (s, 1H, SO₂NHN) ppm.
**MS FAB: m/z(%):** 462 (MH⁺, 58), 154 (100)
**IR (KBr):** 3058, 1621, 1534, 1350, 1266, 1171, 1073, 915, 815, 755, 661, 545 cm⁻¹.
**Elemental analysis: Calculated for C₂₄H₁₉N₃O₅S:** 62.46 %C; 4.15 %H; 9.11 %N.
**Measured:** 63.09 %C, 4.26 %H, 9.00 %N.
**Melting point:** 133-134°C.

### EXAMPLE 25: 2-Formylphenyl-4-nitrobenzenesulfonate

Salycilaldehyde (560 mg (4,586 mmol)) and triethylamine (0,8 mL) were dissolved in 30 mL THF and 4-nitrobenzene-1-sulfonylchloride (1,016 g (4,584 mmol)) was added on icebath. After 3 hours the solvent was removed and the residue dissolved in EtOAc (50 mL) and washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether.
**Yield:** 68.1 %
**Molecular formula:** C₁₃H₉NO₆S
**M**=307.28 g/mol
**¹H-NMR(300 MHz, CDCl₃):**δ 7.20 (dd, J₁=8.18 Hz, J₂=0.87 Hz, 1H, Ar-H), 7.45-7.54 (t, J=7.55 Hz, 1H, Ar-H), 7.60-7.69 (ddd, J₁=8.17 Hz, J₂=7.57 Hz, J₃=1.85 Hz, 1H, Ar-H), 7.91-7.97 (dd, J₁=7.69 Hz, J₂=1.82 Hz, 1H, Ar-H), 8.10-8.16 (d, J_{AB}=8.91 Hz, 2H, Ar-H), 8.40-8.47 (d, J_{AB}=8.90 Hz, 2H, Ar-H), 10.09 (s, 1H, Ar-CHO) ppm.
**MS FAB: m/z(%):** 308 (MH⁺, 100).
**IR (KBr):** 3104, 2869, 1694, 1597, 1531, 1381, 1192, 1089, 899, 804, 603 cm⁻¹.
**Melting point:** 117-119°C.

### EXAMPLE 26: 2-((2-(Naphthylsulfonyl)hydrazono)methyl)phenyl 4-nitrobenzenesulfonate

2-Formylphenyl-4-nitrobenzenesulfonate (200 mg (0,651 mmol)) and triethylamine (0.1mL) were dissolved in THF (10 mL) and 2-naphthalenesulfonohydrazinium chloride (168 mg (0,651 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 80.1 %
**Molecular formula:** C₂₃H₁₇N₃O₇S₂
**M**=511.54g/mol
**¹H-NMR(300 MHz, DMSO-d₆):**δ 7.03-7.09 (dd, J₁=7.99 Hz, J₂=1.22 Hz, 1H, Ar-H), 7.31-7.39 (td, J₁=7.38 Hz, J₂=1.01 Hz, 1H, Ar-H), 7.39-7.46 (td, J₁=7.65 Hz, J₂=2.10 Hz, 1H, Ar-H), 7.63-7.75 (m, 3H, Ar-H), 7.81-7.87 (dd, J₁=8.69 Hz, J₂=1.86 Hz, 1H, Ar-H), 7.90 (s, 1H, Ar-H), 8.00-8.06 (d, J₁=7.49 Hz, 1H, Ar-H), 8.06-8.16 (m, 3H, Ar-H), 8.18-8.24 (dd, J₁=7.26 Hz, J₂=1.76 Hz, 1H, Ar-H), 8.40-8.45 (d, J=8.94 Hz, 2H, Ar-H), 8.55 (s, 1H, Ar-CH=), 11.80 (s, 1H, SO₂NHN=) ppm.
**MS FAB: m/z(%):**512 (MH⁺, 19), 57 (100)
**IR (KBr):** 3203, 1603, 1531, 1358, 1162, 1061, 959, 877, 761 cm⁻¹.
**Elemental analysis: Calculated for C₂₃H₁₇N₃O₇S₂:** 54.01 %C; 3.35 %H; 8.21 %N.
**Measured:** 54.29 %C; 3.52 %H; 8.02 %N.
**Melting point:** 145-148°C.

### EXAMPLE 27: 3-(3-Nitrobenzyloxy)benzaldehyde

3-Hydroxybenzaldehyde (224 mg (1,834 mmol)) and 3-nitrobenzylchloride (315 mg (1,837 mmol)) were dissolved in 10 mL THF. Triethylamine (0,5 mL) and KF (260 mg (4,48 mmol)) were added during stirring and the mixture was refluxed for 12 hours under argon. The solvent was removed under reduced pressure, residue treated with ethylacetate (50 mL) and washed with 10% solution of citric acid (2x10 mL), saturated solution of NaHCO₃ (2x 10 mL), brine (2x 10 mL) and dried over Na₂SO₄. The solvent was evaporated in vacuo and the crude product was recrystallized from diethylether.
**Yield:** 90.3 %
**Molecular formula:** C₁₄H₁₁NO₄
**M**=257.25 g/mol
**¹H-NMR(300 MHz, CDCl₃)**:δ 5.25 (s, 2H, Ar-CH₂), 7.25-7.34 (m, 1H, Ar-H), 7.46-7.56 (m, 3H, Ar-H), 7.57-7.66 (t, J=7.93 Hz, 1H, Ar-H), 7.76-7.84 (d, J=7.35 Hz, 1H, Ar-H), 8.19-8.26 (d, 1H, Ar-H), 8.261 (s, 1H, Ar-H), 10.01 (s, 1H, Ar-CHO) ppm.**MS EI: m/z(%):** 285 (MH⁺, 100)
**MS EI: m/z(%):**257 (M⁺, 20), 136 (100)
**IR (KBr):** 2721, 1692, 1595, 1525, 1345, 1262, 1037, 785, 727, 662.
**Melting point:** 112-115°C

### EXAMPLE 28: N'-({3-[(3-nitrobenzyl)oxy]phenyl}methylidene)-2-naphthalenesulfonohydrazide

3-(3-Nitrobenzyloxy)benzaldehyde (200 mg (0,778 mmol)) and triethylamine (0.15 mL) were dissolved in THF (10 mL) and 2-naphthalenesulfonohydrazinium chloride (201 mg (0,777 mmol)) was added. The reaction mixture was stirred overnight at room temperature, the solvent was evaporated in vacuo and the residue dissolved in 50 mL EtOAc. Organic phase was washed with 10% citric acid (2 × 10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 80.0 %
**Molecular formula:** C₂₄H₁₉N₃O₅S
**M**=461.50 g/mol
**¹H-NMR(300 MHz, DMSO-d₆):δ** 5.14 (s, 2H, Ar-CH₂), 6.95-7.01 (ddd, J₁=8.26 Hz, J₂=2.60 Hz, J₃=0.84 Hz, 1H, Ar-H), 7.11-7.16 (d, J=7.59 Hz, 1H, Ar-H), 7.22-7.32 (m, 1H, Ar-H), 7.52-7.69 (m, 3H, Ar-H), 7.71-7.79 (m, 2H, Ar-H), 7.85-8.01 (m, 5H, Ar-H), 8.17-8.22 (dd, J=8.17 Hz, J₂=1.32 Hz, 1H, Ar-H), 8.31 (s, 1H, Ar-H), 8.58 (s, 1H, Ar-CH=) ppm **MS FAB: m/z(%):**462 (MH⁺, 4), 55 (100)
**IR (KBr):** ν 3226, 1574, 1524, 1354, 1354, 1299, 1164, 1063, 977, 823, 756, 671 cm⁻¹.
**Elemental analysis: Calculated for C₂₄H₁₉N₃O₅S:** 62.46 %C; 4.15 %H; 9.11 %N.
**Measured:** 62.58 %C; 4.19 %H; 9.05 %N.
**Melting point:** 156-158°C.

### EXAMPLE 29: 2-Formylphenyl 2-nitrobenzenesulfonate

Salycilaldehyde (1,109 g (9,08 mmol)) and triethylamine (1,9 mL) were dissolved in 30 mL THF and 2-nitrobenzene-1-sulfonylchloride (2,012 g (9,078 mmol)) was added on icebath. After 3 hours the solvent was removed and the residue dissolved in EtOAc (50 mL) and washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 57.1 %
**Molecular formula:** C₁₃H₉NO₆S
**M**=307.28 g/mol
**¹H-NMR(300 MHz, CDCl₃):**δ 7.35-7.41 (dd, J₁=8.23 Hz, J₂=0.77 Hz, 1H, Ar-H), 7.45-7.53 (t, J=7.55 Hz, 1H, Ar-H), 7.61-7.70 (ddd, J₁=8.24 Hz, J₂=7.57 Hz, J₃=1.83 Hz, 1H, Ar-H), 7.66-7.82 (m, 1H, Ar-H), 7.88-7.93 (m, 2H, Ar-H), 7.93-7.99 (dd, J₁=7.70 Hz, J₂=1.80 Hz, 1H, Ar-H), 7.99-8.05 (d, J=7.69 Hz, 1H, Ar-H), 10.25 (s, 1H, Ar-CHO) ppm.
**MS FAB: m/z(%):** 308 (MH⁺, 22), 55 (100)
**IR (KBr):** 3100, 2884, 2765, 1702, 1604, 1542, 1380, 1194, 1086, 873, 785, 588 cm⁻¹.
**Melting point:** 103-104 °C.

### EXAMPLE 30: 2-((2-(Naphthylsulfonyl)hydrazono)methyl)phenyl 2-nitrobenzenesulfonate

2-Formylphenyl 2-nitrobenzenesulfonate (237 mg (0,771 mmol)) and triethylamine (0.12 mL) were dissolved in THF (10 mL) and 2-naphthalenesulfonohydrazinium chloride (199 mg (0,769 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 45.6 %
**Molecular formula:** C₂₃H₁₇N₃O₇S₂
**M**=511.54 g/mol
**¹H-NMR(300 MHz, DMSO-d₆):** δ 7.03-7.10 (dd, J₁=7.84 Hz, J₂=1.46 Hz, 1H, Ar-H), 7.33-7.48 (m, 2H, Ar-H), 7.66-8.10 (m, 9H, Ar-H), 8.10-8.27 (m, 3H, Ar-H), 8.55 (s, 1H, Ar-CH=), 11.88 (s, 1H, SO₂NHN) ppm.
**MS FAB: m/z(%):** 512 (MH⁺, 45), 154 (100)
**IR (KBr):** ν 3197, 1545, 1364, 1163, 869, 778, 658 cm⁻¹.
**Elemental analysis: Calculated for C₂₃H₁₇N₃O₇S₂:** 54.01 %C; 3.35 %H; 8.21 %N.
**Measured:** 54.18 %C; 3.54 %H; 7.95 %N.
**Melting point:** 68-70°C.

### EXAMPLE 31: 2-Formylphenyl 3-nitrobenzenesulfonate

Salycilaldehyde (1,027 g (8,41 mmol)) and triethylamine (1,8 mL) were dissolved in 30 mL THF and 3-nitrobenzene-1-sulfonylchloride (1,863 g (8,406 mmol)) was added on icebath. After 3 hours the solvent was removed and the residue dissolved in EtOAc (50 mL) and washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The crude product purified by column cromatography (silicagel; hexane:EtOAc = 4:1).
**Yield:** 57.1 %
**Molecular formula:** C₁₃H₉NO₆S
**M**=307.28 g/mol
**¹H-NMR(300 MHz, CDCl₃):**δ 7.21-7.28 (m, 1H, Ar-H), 7.44-7.52 (t, J=7.55 Hz, 1H, Ar-H), 7.60-7.67 (td, J₁=7.80 Hz, J₂=1.80 Hz, 1H, Ar-H), 7.79-7.87 (t, J=8.08 Hz, 1H, Ar-H), 7.88-7.95 (dd, J₁=7.69 Hz, J₂=1.81 Hz, 1H, Ar-H), 8.20-8.27 (ddd, J₁=7.89 Hz, J₂=1.62 Hz, J₃=1.10 Hz, 1H, Ar-H), 8.54-8.61 (ddd, J₁=8.15 Hz, J₂=2.12 Hz, J₃=0.96 Hz, 1H, Ar-H), 8.71-8.76 (t, J=1.93 Hz, 1H, Ar-H), 10.07 (s, 1H, Ar-CHO) ppm.
**MS FAB: m/z(%):** 308 (MH⁺, 14), 55 (100)
**IR (KBr):** 3093, 1694, 1603, 1532, 1350, 1202, 1065, 860, 717, 628, 554 cm⁻¹.
**Elemental analysis: Calculated for C₁₃H₉NO₆S:** 50.81 %C; 2.95 %H; 4.56 %N. Measured: 50.98 %C; 3.01 %H; 4.58 %N.
**Melting point:** 85-86 °C.

### EXAMPLE 32: 2-((2-(Naphthylsulfonyl)hydrazono)methyl)phenyl 3-nitrobenzenesulfonate

2-Formylphenyl 3-nitrobenzenesulfonate (237 mg (0,771 mmol)) and triethylamine (0.2 mL) were dissolved in THF (10 mL) and 2-naphthalenesulfonohydrazinium chloride (179 mg (0,694 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 45.6 %
**Molecular formula:** C₂₃H₁₇N₃O₇S₂
**M**=511.54 g/mol
**¹H-NMR(300 MHz, DMSO-d₆):δ** 7.11-7.18 (dd, J₁=8.04 Hz, J₂=1.22 Hz, 1H, Ar-H), 7.32-7.49 (m, 2H, Ar-H), 7.65-7.75 (m, 3H, Ar-H), 7.77-7.88 (m, 2H, Ar-H), 8.03-8.09 (d, J=7.59 Hz, 1H, Ar-H), 8.10-8.19 (m, 2H, Ar-H), 8.19-8.25 (d, J=7.45 Hz, 1H, Ar-H), 8.35-8.45 (m, 2H, Ar-H), 8.52-8.56 (d, J=1.42 Hz, 1H, Ar-CH=), 11.79 (s, 1H, SO₂NHN=) ppm.
**MS FAB: m/z(%):** 512 (MH⁺, 23), 154 (100)
**IR (KBr): ν** 3165, 2897, 1604, 1527, 1448, 1329, 1194, 1060, 971, 893, 779, 658, 582 cm⁻¹.
**Elemental analysis: Calculated for C₂₃H₁₇N₃O₇S₂:** 54.01 %C; 3.35 %H; 8.21 %N.
**Measured:** 54.24 %C; 3.47 %H; 8.19 %N.
**Melting point:** 145-148°C.

### EXAMPLE 33: 4-((2-Formylphenoxy)methyl)benzonitrile

The mixture of salycilaldehyde (1,328 g (10,875 mmol)) and 2,132 g (10,875 mmol) 4-bromomethylbenzonitrile was dissolved in acetone and K₂CO₃ (2,00 g (14,47 mmol)) was added during the stirring. The mixture was refluxed for 12 hours and the solvent was removed in vacuo. EtOAc (100 mL) was added to the solid residue and washed with with 10% citric acid (2×20 mL), aqueous solution of NaHCO₃ (2×20 mL), purified water (2×20 mL) and brine (2×20 mL). Organic phase was dried with Na₂SO₄ and the solvent evaporated in vacuo. The product was recrystallized from diethylether.
**Yield**: 75.6%
**Molecular formula:** C₁₅H₁₁NO₂
**M**=237.26 g/mol
**¹H-NMR(300 MHz, CDCl₃):** δ 5.19 (s, 2H, Ar-CH₂), 7.22-7.28 (m, 1H, Ar-H), 7.44-7.52 (m, 3H, Ar-H), 7.53-7.59 (d, J_{AB}=8.38 Hz, 2H, Ar-H), 7.67-7.73 (d, J_{AB}=8.31 Hz, 2H, Ar-H), 9.98 (s, 1H, Ar-CHO) ppm.
**MS FAB: m/z(%):** 237 (MH⁺, 49), 116 (100)
**IR (KBr):** 2223, 1692, 1598, 1484, 1395, 1264, 1166, 1049, 866, 783, 680, 543 cm⁻¹.
**Melting point:** 99-100 °C

### EXAMPLE 34: N'-(2-(4-Cianobenziloksi)benziliden)-naftalen-2-sulfonohidrazid

4-((2-Formylphenoxy)methyl)benzonitrile (237 mg (1,000 mmol)) and triethylamine (0.15 mL) were dissolved in THF (10 mL) and 2-naphthalenesulfonohydrazinium chloride (259 mg (1,000 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 64.1 %
**Molecular formula:** C₂₅H₁₉N₃O₃S
**M**=441.51 g/mol
**¹H-NMR(300 MHz, DMSO-d₆):**δ5.12 (s, 1H, Ar-CH₂), 6.77-6.87 (m, 2H, Ar-H), 7.19-7.27 (td, J₁=7.73 Hz, J₂=1.80 Hz, 1H, Ar-H), 7.44-7.51 (dd, J₁=7.98 Hz, J₂=1.59 Hz, 1H, Ar-H), 7.55-7.81 (d, J=8.32 Hz, 2H, Ar-H), 7.68-7.80 (m, 2H, Ar-H), 7.84-7.97 (m, 4H, Ar-H), 8.05-8.11 (d, J=7.50 Hz, 1H, Ar-H), 8.18-8.27 (t, J=8.65 Hz, 2H, Ar-H), 8.66-8.71 (d, J=1.50 Hz, 1H, Ar-CH=), 10.17 (s, 1H, CONHN=) ppm.
**MS FAB: m/z(%):** 442 (MH⁺, 52), 154 (100)
**IR (KBr):** ν 3077, 2226, 1609, 1485, 1348, 1267, 1159, 1046, 899, 759, 656 cm⁻¹.
**Elemental analysis: Calculated for C₂₅H₁₉N₃O₃S:** 68.24 %C; 4.32 %H; 9.54 %N.
**Measured:** 68.01 %C; 4.34 %H; 9.52 %N.
**Melting point:** 147-150°C.

### EXAMPLE 35: 2-(2-Nitrobenzyloxy)benzaldehyde

2-Hydroxybenzaldehyde (606 mg (4,962 mmol)) and 2-nitrobenzylchloride (851 mg (4,962 mmol)) were dissolved in THF (20 mL). Triethylamine (0,9 mL) and KF (577 mg (9,931 mmol)) were added during stirring and the mixture was refluxed for 12 hours under argon. The solvent was removed under reduced pressure, residue treated with ethylacetate (50 mL) and washed with 10% solution of citric acid (2x10 mL), saturated solution of NaHCO₃ (2x10 mL), brine (10 mL) and dried over Na₂SO₄. The solvent was evaporated in vacuo and the crude product purified by column cromatography (silicagel; hexane:EtOAc = 4:1).
**Yield:** 12.5%
**Molecular formula:** C₁₄H₁₁NO₄
**M**=257.25 g/mol
**¹H-NMR(300 MHz, DMSO-d₆):**δ 5.45 (s, 1H, Ar-CH₂), 7.09-7.17 (t, J=7.49 Hz, 1H, Ar-H), 7.30-7.37 (d, J=8.38 Hz, 1H, Ar-H), 7.64-7.72 (m, 1H, Ar-H), 7.72-7.78 (d, J₁=7.78 Hz, J₂=2.18 Hz, 2H, Ar-H), 7.97-8.05 (d, J=7.71 Hz, 1H, Ar-H). 8.18-8.25 (dd, J₁=8.10 Hz, J₂=1.57 Hz, 1H, Ar-H), 8.40 (s, 1H, Ar-H), 10.45 (s, 1H, Ar-CHO) ppm.
**MS FAB: m/z(%):** 258 (MH⁺, 33), 136 (100)
**IR (KBr):** 3452, 1679, 1599, 1528, 1461, 1350, 1238, 1025, 802, 730 cm⁻¹.
**Elemental analysis: Calculated for C₁₄H₁₁NO₄:** 65.37 %C; 4.31 %H; 5.44 %N. Measured: 64.99 %C; 4.16 %H; 5.46 %N.
**Melting point:** 103-105°C.

### EXAMPLE 36: N'-(2-(2-Nitrobenzyloxy)benzylidene)naphthalene-2-sulfonohydrazide

2-(2-Nitrobenzyloxy)benzaldehyde (145 mg (0,564 mmol)) and triethylamine (0.1 mL) were dissolved in THF (10 mL) and 2-naphthalenesulfonohydrazinium chloride (146 mg (0,564 mmol)) was added. The reaction mixture was stirred overnight at room temperature, the solvent was evaporated in vacuo and the residue dissolved in 50 mL EtOAc. Organic phase was washed with 10% citric acid (2×10 mL), aqueous solution of NaHCO₃ (2x10 mL), purified water (2x10 mL) and brine (2×10 mL). Organic phase was dried with Na₂SO₄ and the solvent evaporated in vacuo. The product was recrystallized from diethylether/EtOAc.
**Yield:** 68.8 %
**Molecular formula:** C₂₄H₁₉N₃O₅S
**M**=461.50g/mol
**¹H-NMR(300 MHz**, **DMSO-d₆):**δ 5.47 (s, 2H, Ar-CH₂-), 6.94-7.02 (t, J=7.51 Hz, 1H, ArH), 7.07-7.13 (d, J=8.15 Hz, 1H, Ar-H), 7.30-7.40 (dd, J₁=7.27 Hz, J₂=1.57 Hz, 1H, Ar-H), 7.59-7.75 (m, 5H, Ar-H), 7.77-7.82 (d, J=4.20 Hz, 2H, Ar-H), 7.84-7.91 (dd, J₁=8.68 Hz, J₂=1.87 Hz, 1H, Ar-H), 7.99-8.05 (d, J=7.50 Hz, 1H, Ar-H), 8.09-8.23 (m, 3H, Ar-H), 8.29 (s, 1H, Ar-H), 8.56 (s, 1H, Ar-CH=), 11.603 (s, 1H, Ar-H) ppm.
**MS FAB: m/z(%):** 462 (MH⁺, 17), 154 (100)
**IR (KBr):** ν 3491, 3225, 1603, 1519, 1329, 1246, 1159, 1049, 954, 745, 659, 544 cm⁻¹.
**Elemental analysis: Calculated for C₂₄H₁₉N₃O₅S:** 62.46 %C; 4.15 %H; 9.11 %N.
**Measured:** 62.62 %C; 4.20 %H; 9.05 %N.
**Melting point:** 174-179°C.

### EXAMPLE 37: 3-Nitrophenylsulfonohydrazide

To the solution of m-nitrophenylsulfonyl chloride (2,00 g, (9,02 mmol)) in THF (20 ml) hydrazine monohydrate (1,1 mL) was added at -15°C. The mixture was stirred for 30 minutes and EtOAc (60 mL) was added. Organic phase was washed with brine (3×30 mL). Organic phase was dried with Na₂SO₄ and hexane (200 ml) was slowly added. After 10 minutes a white precipitate formed that was filtered off and dried on air.
**Yield:** 78.0 %
**Molecular formula:** C₆H₂₂N₃O₄S
**M**=217.20 g/mol
**¹H NMR (300 MHz, DMSO-d₆):** δ 4.33 (s, 2H, NH₂), 7.91 (t, 1H, J=8,0 Hz), 8.17-8.23 (m, 1H, Ar-H), 8.48-8.56 (m, 2H, Ar-H), 8.72 (s, 1H, NH) ppm.
**MS (FAB): m/z (%):** 218 (MH⁺, 80), 154 (100).
**IR (KBr):** ν 3353, 3263, 1608, 1527, 1355, 1171, 1073, 886, 736, 664, 574 cm⁻¹.
**Melting point:** 131-133°C.

### EXAMPLE 38: N'-(2-(4-Cyanobenzyloxy)benzylidene)-4-trifluoromethylbenzyl-1-sulfonohydrazide

4-Trifluorobenzylsulfonohydrazide (230 mg (0,905 mmol)) and 2-(4-cyanobenzyloxyi)benzaldehyde (215 mg (0,906 mmol)) were dissolved in absolute alcohol (30 mL) and stirred at 40°C for 3h. The mixture was cooled and the precipitate was collected on filter, washed with diethyether and dried on air.
**Yield:** 79.2%
**Molecular formula:** C₂₃H₁₈N₃O₃SF₃
**M**=473.48 g/mol
**¹H NMR (300 MHz, DMSO-d₆):** δ 4.72 (s, 2H, Ar-CH₂-O), 5.31 (s, 2H, Ar-CH₂-SO₂), 7.04 (t, 1H, J=7.5 Hz, Ar-H), 7.18 (d, 1H, J=8,3 Hz, Ar-H), 7.40-7.46 (m, 1H, Ar-H), 7.56 (d, 2H, J=8.1 Hz, Ar-H), 7.67-7.79 (m, 6H, Ar-H), 7.88 (d, 2H, J=8.3 Hz, Ar-H), 8.38 (s, 111, CH=N), 11.30 (s, 1H, NH) ppm.
**MS (FAB): m/z (%):** 474 (MH⁺, 25), 154 (100).
**IR (KBr): ν** 3191, 2226, 1605, 1452, 1325, 1158, 960, 849, 755, 647 cm⁻¹.
**Elemental analysis: Calculated for C₂₃H₁₈N₃O₃SF₃:** 58.35 %C; 3.83 %H; 8.87 %N.
**Measured:** 58.44 %C; 4.07 %H; 8.80 %N.
**Melting point:** 190-192°C.

### EXAMPLE 39: N'-(2-(4-Cyanobenzyloxy)benzylidene)-2-fluorobenzyl-1-sulfonohydrazide

2-Fluorobenzylsulfonohydrazide (200 mg (0,978 mmol)) and 2-(4-cyanobenzyloxyi)benzaldehyde (232 mg (0,978 mmol)) were dissolved in absolute alcohol (30 mL) and stirred at 40°C for 3h. The mixture was cooled and the precipitate was collected on filter, washed with diethyether and dried on air.
**Yield:** 75.0%
**Molecular formula:** C₂₂H₁₈N₃O₃SF
**M**=423.47 g/mol
**¹H NMR (300 MHz, DMSO-d₆):** δ 4.59 (s, 2H, Ar-CH₂-O), 5.32 (s, 2H, Ar-CH₂-SO₂), 7.05 (t, 1H, J=7.5 Hz, Ar-H), 7.12-7.24 (m, 3H, Ar-H), 7.42 (ddd, 3H, J₁=1.9 Hz, J₂=4.1 Hz, J₃=8.0 Hz, Ar-H), 7,69 (d, 2H, J=8.2 Hz, Ar-H), 7.77 (dd, 1H, J₁=1.5 Hz, J₂=7.7 Hz, Ar-H), 7.89 (d, 2H, J=8.2 Hz, Ar-H), 8.40 (s, 1H, CH=N), 11.35 (s, 1H, NH) ppm.
**MS (FAB): m/z (%):** 424 (MH⁺, 40), 154 (100).
**IR (KBr):** ν 3151, 2226, 1600, 1451, 1324, 1246, 1045, 955, 757, 555 cm⁻¹.
**Elementna analiza: Izra** 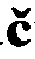 **uno za C₂₂H₁₈N₃O₃SF:** 62.40 %C; 4.28 %H; 9.92 %N. **Measured:** 62.44 %C; 4.39 %H; 9.88 %N.
**Melting point:** 179-181°C.

### EXAMPLE 40: N'-(2-(4-Cyanobenzyloxy)benzylidene)-benzyl-1-sulfonohydrazide

Benzylsulfonohydrazide (100 mg (0,537 mmol)) and 2-(4-cyanobenzyloxyi)benzaldehyde (130 mg (5,48 mmol)) were dissolved in absolute alcohol (30 mL) and stirred at 40°C for 3h. The mixture was cooled and the precipitate was collected on filter, washed with diethyether and dried on air.
**Yield**: 71.4%
**Molecular formula:** C₂₂H₁₉N₃O₃S
**M**=405.48 g/mol
**¹H NMR (300 MHz, DMSO-d₆):** δ 4.56 (s, 2H, Ar-CH₂-O), 5.32 (s, 2H, Ar-CH₂-SO₂), 7.06 (t, 1H, J=7.5 Hz, Ar-H), 7.19 (d, 1H, J=8.4 Hz, Ar-H), 7.31-7.45 (m, 6H, Ar-H), 7.69 (d, 2H, J=8.1 Hz, Ar-H), 7.84 (dd, 1H, J₁=1.4 Hz, J₂=7.8 Hz, Ar-H), 7.89 (d, 2H, J=8.1 Hz, Ar-H), 8.40 (s, 1H, CH=N), 11.25 (s, 1H, NH) ppm.
**MS (FAB): m/z** (%): 406 (MH⁺, 55), 154 (100).
**IR (KBr):** ν 3147, 2229, 1602, 1449, 1327, 1251, 1048, 953, 821, 695, 540 cm⁻¹.
**Elemental analysis: Calculated for C₂₂H₁₉N₃O₃S:** 65.17 %C; 4.72 %H; 10.36%N. **Measured:** 65.12 %C; 4.90 %H; 10.38 %N.
**Melting point:** 175-177°C.

### EXAMPLE 41: N'-(2-(4-Cyanobenzyoxy)benzylidene)-3-nitrophenyl-1-sulfonohydrazide

3-Nitrobenzylsulfonohydrazide (500 mg (2,16 mmol)) and 2-(4-cyanobenzyloxyi)benzaldehyde (546 mg (2,30 mmol)) were dissolved in absolute alcohol (30 mL) and stirred at 40°C for 3h. The mixture was cooled and the precipitate was collected on filter, washed with diethyether and dried on air.
**Yield**: 80.0%
**Molecular formula:** C₂₁H₁₆N₄O₅S
**M** = 436.45 g/mol
**¹H NMR (300 MHz, DMSO-d₆):** δ 5.26 (s, 2H, Ar-CH₂-O), 7.00 (t, 1H, J=7.5 Hz, Ar-H), 7.13 (d, 1H, J=8.1 Hz, Ar-H), 7.36-7.42 (m, 1H, Ar-H), 7.63-7.68 (m, 3H, Ar-H), 7.86-7.96 (m, 3H, Ar-H), 8.28-8.31 (m, 1H, Ar-H), 8.35 (s, 1H, CH=N), 8.49 (ddd, 1H, J₁=1.0 Hz, J₂=2.3 Hz, J₃=8.3 Hz, Ar-H), 8.58 (t, 1H, J=1.9 Hz, Ar-H), 11.79 (s, 1H, NH) ppm.
**MS (FAB): m/z (%):** 437 (MH⁺, 25), 55 (100).
**IR (KBr):** ν 3222, 3091, 2233, 1603, 1531, 1340, 1174, 1052, 817, 759, 668cm⁻¹.
**Elemental analysis: Calculated for C₂₁H₁₆N₄O₅S:** 57.79%C; 3.70 %H; 12.84 %N. **Measured:** 57.99 %C; 3.92 %H; 12.72 %N.
**Melting point:** 185-187°C.

### EXAMPLE 42: N'-(2-(4-Cyanobenzyloxy)benzylidene)-2-nitrophenyl-1-sulfonohydrazide

2-Nitrobenzylsulfonohydrazide (300 mg (1,30 mmol)) and 2-(4-cyanobenzyloxyi)benzaldehyde (328 mg (1,38 mmol)) were dissolved in absolute alcohol (30 mL) and stirred at 40°C for 3h. The mixture was cooled and the precipitate was collected on filter, washed with diethyether and dried on air.
**Yield:** 74.5%
**Molecular formula:** C₂₁H₁₆N₄O₅S
**M**=436.45 g/mol
**¹H NMR (300 MHz, DMSO-d₆):** δ 5.29 (s, 2H, Ar-CH₂-O), 6.99 (t, 1H, J=7.5 Hz, Ar-H), 7.15 (d, 1H, J=8.3 Hz, Ar-H), 7.37-7.42 (m, 1H, Ar-H), 7.63-7.69 (m, 3H, Ar-H), 7.87-7.91 (m, 4H, Ar-H), 7.99-8.07 (m, 2H, Ar-H), 8.50 (s, 1H, CH=N), 12.08 (s, 1H, NH) ppm.
**MS (FAB): m/z (%):** 437 (MH⁺, 35), 154 (100).
**IR (KBr):** ν 3235, 2232, 1599, 1524, 1452, 1371, 1240, 1173, 1032, 953, 758, 658, 589 cm⁻¹.
**Elemental analysis: Calculated for C₂₁H₁₆N₄O₅S:** 57.79 %C; 3.70 %H; 12.84 %N.
**Measured:** 57.64 %C; 3.88 %H; 12.96 %N
**Melting point:** 207-210°C

### EXAMPLE 43: Methyl 2-(3-((2-(naphthalene-2-ylsulfonyl)hydrazono)methyl)phenoxy) benzoate

2-(3-Formylphenoxy)benzoate (97 mg (0,379 mmol)) and triethylamine (0.1mL) were dissolved in THF (10 mL) and 2-naphthalenesulfonohydrazinium chloride (97.6 mg (0.378 mmol)) was added. The reaction mixture was stirred overnight at room temperature, the solvent was evaporated in vacuo and the residue dissolved in 50 mL EtOAc. Organic phase was washed with 10% citric acid (2 × 10 mL), aqueous solution of NaHCO₃ (2×10 mL), purified water (2×10 mL) and brine (2×10 mL). Organic phase was dried with Na₂SO₄ and the solvent evaporated in vacuo. The product was recrystallized from diethylether/EtOAc.
**Yield:** 63.1 %
**Molecular formula:** C₂₅H₂₀N₂O₅S
**M**=460.51g/mol
**¹H-NMR(300 MHz, DMSO-d₆):**δ 3.65 (s, 3H, COOCH₃), 6.90-6.96 (dd, *J₁* = 7.62 Hz, *J₂*=1.99 Hz, 1H, Ar-H), 7.03-7.10 (m, 2H, Ar-H), 2.24-7.31 (d, J=7.70 Hz, 1H, Ar-H), 7.31-7.40 (t, J=7.71 Hz, 2H, Ar-H), 7.56-7.65 (td, J₁=8.15 Hz, J₂=1.69 Hz, 1H, Ar-H), 7.65-7.76 (m, 2H, Ar-H), 7.76-7.83 (dd, J₁=8.68 Hz, J₂=1.75 Hz, 1H, Ar-H), 7.85-7.92 (m, 2H, Ar-H), 8.01-8.07 (d, J=7.56 Hz, 1H, Ar-H), 8.07-8.12 (d, J=8.74 Hz, 1H, Ar-H), 8.13-8.20 (d, J=7.54 Hz, 1H, Ar-H), 8.50 (s, 1H, Ar-CH=), 11.63 (s, 1H, SO₂NHN=) ppm.
**MS FAB: m/z(%):**461 (MH⁺, 85), 154 (100)
**IR (KBr):** ν 3145, 1722, 1571, 1482, 1360, 1253, 1158, 1046, 917, 749, 686, 545 cm⁻¹.
**Elemental analysis: Calculated for C₂₅H₂ON₂O₅S:** 65.21 %C; 4.38 %H; 6.08 %N.
**Measured:** 64.97 %C; 4.46 %H; 6.20 %N.
**Melting point:** 151-155°C

### EXAMPLE 44: 2-{[2-(2-Naphthylsulfonyl)hydrazono]methyl}benzoic acid

2-Formilbenzoic acid (2.00 g (13.3 mmol)) was dissolved in absolute alcohol (50 mL) and 2-naphthalenesulfonohydrazinium chloride ((3.45 g (13.3 mmol)) and triethylamine (1,0 mL) were added . The mixture was stirred for 24 hours and the solvent evaporated in vacuo. The residue was dissolved in methanol (15 mL) and diethylether was added dropwise until the precipitate formed. The produkt was filtered off and dried on air.
**Yield:** 87.8%
**Molecular formula:** C₁₈H₁₄N₂O₄S
**M:** 354.381 gmol⁻¹
**¹H NMR (300 MHz, DMSO-d6):** δ 7.46 (t, 1H, J=7,52 Hz, Ar-H), 7.55 (t, 1H, J=7.51 Hz, Ar-H), 7.70 (t, 2H, J=6.38 Hz, Ar-H), 7.76 (d, 1H, J=7.93 Hz, Ar-H), 7.83 (dd, 1H, J₁=7.75 Hz, J₂=1.17 Hz, Ar-H), 7.89 (dd, 1H, J₁=8.69 Hz, J₂=1.84, Ar-H), 8.02-8.05 (m, 2H, Ar-H), 8.23 (d, 1H, J=7.19 Hz, Ar-H), 8.15 (d, 1H, J=8.78 Hz, Ar-H), 8.58 (s, 1H, N=CH-Ar), 8.70 (s, 1H, SO₂-NH-N), 11.78 (S, 1H, COOH) ppm
**MS (FAB): m/z(%):** 355 (MH⁺, 68), 102 (100)
**IR (KBr):** 3379, 3059, 1576, 1380, 1330, 1166, 1053, 965, 816, 766, 656, 550 cm⁻¹
**Elemental analysis: Calculated for C₁₈H₁₄N₂O₄S:** 61.01 %C, 3.98 %H, 7.90 %N. **Measured:** 61.28 %C, 4.23 %H, 8.93 %N.
**Melting point:** 119-120°C

### General procedure for the synthesis of compounds with X = -CONH- (EXAMPLES 45 - 54)

2-{[(2-(2-Naphthylsulfonyl)hydrazono]methyl}benzoic acid (1.50 g (4.23 mmol)) was dissolved in 25 mL CH₂Cl₂, 5.08 mmol of reagent with an amino functional group, EDC (0,81 g (4,23 mmol)), HOBT (0,57 g (4.23 mmol)) and 1mL of triethylamine were added. The mixture was stirred for 24 hours. Organic phase was washed with 10% citric acid (2×10 mL), aqueous solution of NaHCO₃ (2×10 mL), purified water (2x10 mL) and brine (2×10 mL). Organic phase was dried with Na₂SO₄ and the solvent evaporated in vacuo. The product was recrystallized from ethanol.

### EXAMPLE 45: N-(2,4-Dimethylphenyl)-2-{[2-(2-naphthylsulfonyl)hydrazono]methyl} benzamide

**Yield:** 38.8%
**Molecular formula:** C₂₆H₂₃N₃O₃S
**M:** 457.545 gmol⁻¹
**¹H NMR (300 MHz, DMSO-d6):** δ 2.01 (s, 3H, CH₃), 2.09 (s, 3H, CH₃), 5.54 (d, 1H, J=8.93 Hz, CH=N), 6.21 (d, 1H, J=7.82 Hz, Ar-H), 6.37 (t, 1H, J=7.54 Hz, Ar-H), 6.51 (m, 1H, Ar-H), 6.70 (t, 1H, J=7.13 Hz, Ar-H), 7.47 (m, 2H, Ar-H), 7.61-7.71 (m, 4H, Ar-H), 7.84 (d, 1H, J=8.06 Hz, Ar-H), 8.03 (t, 3H, J=8,00 Hz, Ar-H), 8.34 (s, 1H, CO-NH), 10.61 (s, 1H, =N-NH-SO₂) ppm
**MS (EI): m/z(%):** 457 (M⁺, 17), 121 (100)
**IR (KBr):** 3414, 1725, 1618, 1518, 1344, 1161, 1074, 748, 655, 546 cm⁻¹
**Elemental analysis: Calculated for C₂₆H₂₃N₃O₃S:** 68.25 %C, 5.07 %H, 9.18 %N. **Measured:** 68.01 %C, 5.33 %H, 8.84 %N.
**Melting point:** 204-206 °C

### EXAMPLE 46: N-(2,3-Dimethylphenyl)-2-{[2-(2-naphthylsulfonyl)hydrazono]methyl} benzamide

**Yield:** 35.7%
**Molecular formula:** C₂₆H₂₃N₃O₃S
**M:** 457.545 gmol⁻¹
**¹H NMR (300 MHz, DMSO-d6):** δ 1.89 (s, 3H, CH₃), 2.10 (s, 3H, CH₃), 5.65 (d, 1H, J=8.83 Hz, CH=N), 6.24 (s, 1H, Ar-H), 6.41 (q, 2H, J=7.48 Hz, Ar-H), 6.64 (t, 1H, J=7.74 Hz, Ar-H), 7.48 (m, 2H, Ar-H), 7.61-7.70 (m, 4H, Ar-H), 7.82 (d, 1H, J=8.44 Hz, Ar-H), 8.00 (t, 3H, J=7.56 Hz, Ar-H), 8.33 (s, 1H, CO-NH), 10.61 (s, 1H, =N-NH-SO₂) ppm
**MS (FAB): m/z(%):** 458 (MH⁺, 66), 154 (100)
**IR (KBr):** 3454, 3068, 2898, 1702, 1590, 1480, 1348, 1157, 1085, 988, 824, 740, 540 cm⁻¹
**Elemental analysis: Calculated for C₂₆H₂₃N₃O₃S:** 68.25 %C, 5.07 %H, 9.18 %N. **Measured:** 68.36 %C, 5.11 %H, 9.52 %N.
**Melting point:** 120-123 °C

### EXAMPLE 47: N-(1,3-Benzothiazol-2-yl)-2-{(2-(2-naphthylsulfonyl)hydrazono)methyl} benzamide

**Yield**: 33.0%
**Molecular formula:** C₂₅H₁₈N₄O₃S₂
**M:** 486.568 gmol⁻¹
**¹H NMR (300 MHz, DMSO-d6):** δ 5.76 (s, 1H, CH=N), 7.45 (t, 2H, J=7.78 HZ, Ar-H), 7.53 (m, 2H, Ar-H), 7.62-7.72 (m, 4H, Ar-H), 7.73-7.78 (m, 3H, Ar-H), 7.86 (d, 1H, J=8.45 Hz, Ar-H), 8.05 (t, 3H, J=7.52 Hz, Ar-H), 9.54 (s, 1H, CO-NH), 10.94 (s, 1H, =N-NH-SO₂) ppm
**MS (FAB): m/z(%):** 487 (MH⁺, 24), 55 (100)
**IR (KBr):** 3342, 2922, 1591, 1501, 1447, 1245, 1213, 1093, 1015, 867, 746, 715 cm⁻¹
**Elemental analysis: Calculated for C₂₅H₁₈N₄O₃S₂ × 2/3H₂O:** 60.22 %C, 3.91 %H, 11.24 %N. **Measured**: 60.43 %C, 4.05 %H, 11.10 %N.
**Melting point:** 150-155°C

### EXAMPLE 48: N-(Benzimidazol-2-yl)-2-{[2-(2-naphthylsulfonyl)hydrazono]methyl} benzamide

**Yield**: 37.8%
**Molecular formula:** C₂₅H₁₉N₅O₃S
**M:** 469.516 gmol⁻¹
**¹H NMR (300 MHz, DMSO-d6):** δ 5.75 (s, 1H, CH=N), 7.11 (m, 1H, Ar-H), 7.20 (m, 1H, Ar-H), 7.33 (t, 1H, J=6.99 Hz, Ar-H), 7.51 (t, 1H, J=6.89 Hz, Ar-H), 7.70-7.79 (m, 4H, Ar-H), 7.82 (d, 1H, J=7.89 Hz, Ar-H), 7.89 (d, 1H, J=8.83 Hz, Ar-H), 8.03 (t, 3H, J=7.79 Hz, Ar-H), 8.15 (d, 1H, J=8.80 Hz, Ar-H), 8.23 (d, 1H, J=8.94 Hz, Ar-H), 8.90 (s, 1H, CO-NH), 11.00 (s, 1H, =N-NH-SO₂), 12.13 (s, 1H, Ar-NH) ppm
**MS (FAB): m/z(%):** 469 (MH⁺, 31), 134 (100)
**IR (KBr):** 3047, 1729, 1680, 1478, 1348, 1157, 1092, 1026, 808, 738 cm⁻¹
**Elemental analysis: Calculated for C₂₅H₁₉N₅O₃S:** 63.95 %C, 4.08 %H, 14.92 %N. **Measured:** 63.67 %C, 4.36 %H, 14.83 %N.
**Melting point:** 160-165 °C

### EXAMPLE 49: N-(1,3-Thiazol-2-yl)-2-{[2-(2-naphthylsulfonyl)hydrazono]methyl} benzamide

**Yield:** 48.3%
**Molecular formula:** C₂₁H₁₆N₄O₃S₂
**M:** 436.509 gmol⁻¹
**¹H NMR (300 MHz, DMSO-d6):** δ 5.43 (s, 1H, CH=N), 6.57 (d, 1H, J=4.79 Hz, Ar-H), 6.90 (m, 2H, Ar-H), 7.49 (m, 2H, Ar-H), 7.59-7.72 (m, 4H, Ar-H), 7.87 (d, 1H, J=8.52 Hz, Ar-H), 8.03 (t, 3H, Ar-H), 10.92 (s, 1H, CO-NH), 12.34 (s, 1H, =N-NH-SO₂) ppm
**MS (FAB): m/z(%):** 437 (MH⁺, 53), 337 (100)
**IR (KBr):** 3338, 2922, 1587, 1498, 1439, 1248, 1093, 1019, 914, 859, 778, 746, 618 cm⁻¹
**Elemental analysis: Calculated for C₂₁H₁₆N₄O₃S₂:** 57.78 %C, 3.69 %H, 12.84 %N. **Measured:** 57.35 %C, 3.88 %H, 12.59 %N.
**Melting point:** 203-207 °C

### EXAMPLE 50: N-[4-(Aminosulfonyl)phenylethyl]-4-{[(2-(2-naphthylsulfonyl) hydrazono]methyl}benzamide

Yield: 12.4%
**Molecular formula:** C₂₆H₂₄N₄O₅S₂
**M:** 536.625 gmol⁻¹
**1H NMR (300 MHz, DMSO-d6):** δ 2.94 (t, 2H, J=7.10 Hz, CH₂-CH₂-Ar), 3.50 (q, 2H, J=6.95 Hz, NH-CH₂), 7.70 (m, 4H, Ar-H), 7.36-7.56 (m, 3H, Ar-H), 7.79-7.99 (m, 4H, Ar-H), 8.05 (d, 2H, J= 8.65 Hz, Ar-H), 8.13 (d, 2H, J=8.88 Hz, Ar-H), 8.40 (s, 1H, CO-NH), 8.47 (m, 2H, SO₂-NH₂), 8.58 (s, 1H, CH=N), 11.90 (s, 1H, =N-NH-SO₂) ppm
**MS (EI): m/z(%):** 536 (M⁺, 37), 177 (100)
**IR (KBr):** 3433, 1636, 1540, 1329, 1163, 1094, 828, 743, 677 cm⁻¹
**Elemental analysis: Calculated for C₂₆H₂₄N₄O₅S₂:** 58.19 %C, 4.51 %H, 10.44 %N.
**Measured:** 58.35 %C, 4.38 %H, 10.23 %N.
Melting point: **199-205 °C**

### EXAMPLE 51: N-(4-Cyanophenyl)-4-{[2-(2-naphthylsulfonyl)hydrazono]methyl} benzamide

Yield: 13.3%
**Molecular formula:** C₂₅H₁₈N₄O₃S
**M**: 454.502 gmol⁻¹
**¹H NMR (300 MHz, DMSO-d6):** δ 7.69 (m, 3H, Ar-H), 7.75-7.85 (m, 4H, Ar-H), 7.89-8.05 (m, 4H, Ar-H), 8.08 (d, 2H, J=8.16 Hz, Ar-H), 8.22 (d, 2H, J=8.03 Hz, Ar-H), 8.77 (s, 1H, CH=N), 10.63 (s, 1H, CO-NH), 11.80 (s, 1H, =N-NH-SO₂) ppm
**MS (FAB): m/z(%):** 455 (MH⁺, 57), 55 (100)
**IR (KBr):** 3461, 2222, 1660, 1511, 1319, 836, 548 cm⁻¹
**Elemental analysis: Calculated for C₂₅H₁₈N₄O₃S:** 66.07 %C, 3.99 %H, 12.33 %N.
**Measured:** 65.73 %C, 4.15 %H, 12.12 %N.
**Melting point:** 201-205 °C

### EXAMPLE 52: N-(2,4-Dimethylphenyl)-4-{[2-(2-naphthylsulfonyl)hydrazono]methyl} benzamide

**Yield:** 13.3%
**Molecular formula:** C₂₆H₂₃N₃O₃S
**M**: 457.545 gmol⁻¹
**¹H NMR (300 MHz, DMSO-d6):** δ 2.20 (s, 3H, CH₃), 2.25 (s, 3H, CH₃), 7.04 (m, 1H, Ar-H), 7.47 (m, 2H, Ar-H), 7.67 (m, 3H, Ar-H), 8.00 (m, 5H, Ar-H), 8.13 (m, 2H, Ar-H), 8.49 (d, 1H, H=6.10 Hz, Ar-H), 8.60 (s, 1H, CH=N), 9.81 (s, 1H, CO-NH), 11.82 (s, 1H, =N-NH-SO₂) ppm
**MS (FAB): m/z(%):** 458 (MH⁺, 70), 71 (100)
**IR (KBr):** 3291, 3199, 1646, 1501, 1323, 1160, 1065, 969, 814, 674 cm⁻¹
**Elemental analysis: Calculated for C₂₆H₂₃N₃O₃S:** 68.25 %C, 5.07 %H, 9.18 %N.
**Measured:** 67.92 %C, 5.32 %H, 8.76 %N.
**Melting point:** 195-198 °C

### EXAMPLE 53: N-(1,3-Benzothiazol-2-yl)-4-{[(2-(2-naphthylsulfonyl)hydrazono]methyl} benzamide

**Yield:** 30.3%
**Molecular formula: C₂₅H₁₈N₄O₃S₂**
**M:** 486.568 gmol⁻¹
**¹H NMR (300 MHz, DMSO-d6):** δ 7.34 (t, 1H, J=7.15 Hz, Ar-H), 7.47 (t, 1H, J=7.66 Hz, Ar-H), 7.83 (dd, 1H, J₁= 8.63 Hz, J₂= 1.81 Hz, Ar-H), 7.92 (dd, 1H, J₁= 8.70 Hz, J₂= 1.81 Hz, Ar-H), 7.63-1.78 (m, 7H, Ar-H), 8.02 (d, 2H, J=7.77 Hz, Ar-H), 8.24 (d, 2H, J=8.90 Hz, Ar-H), 8.62 (s, 1H, CH=N), 11.94 (s, 1H, =N-NH-SO₂), 12.77 (s, 1H, CO-NH) ppm
**MS (FAB): m/z(%):** 487 (MH⁺, 43), 55 (100)
**IR^{a} (KBr):** 3338, 2918, 1591, 1501, 1443, 1346, 1217, 1085, 1015, 867, 711 cm⁻¹
**Elemental analysis: Calculated for C₂₅H₁₈N₄O₃S₂:** 61.71 %C, 3.73 %H, 11.51 %N. **Measured:** 61.98 %C, 3.81 %H, 11.29 %N.
**Melting point:** 141-143 °C

### EXAMPLE 54: 4-{[2-(2-Naphthylsulfonyl)hydrazono]methyl}-N-[3-(trifluoromethyl)phenyl] benzamide

### Yield: 25.1%

**Molecular formula:** C₂₅H₁₈F₃N₃O₃S
**M:** 497.490 gmol⁻¹
**¹H NMR (300 MHz, DMSO-d6):** δ 7.53 (m, 3H, Ar-H), 7.68 (m, 4H, Ar-H), 7.42 (d, 2H, J=7.71 Hz, Ar-H), 7.78 (d, 2H, J=8.28 Hz, Ar-H), 8.06-8.16 (m, 4H, Ar-H), 8.49 (s, 1H, CH=N), 10.55 (s, 1H, CO-NH), 11.92 (s, 1H, =N-NH-SO₂) ppm
**MS (FAB): m/z(%):** 498 (MH⁺, 10), 57 (100)
**IR^{a} (KBr):** 3342, 2922, 1583, 1501, 1439, 1213, 1089, 910, 754, 715 cm⁻¹
**Elemental analysis: Calculated for C₂₅H₁₈F₃N₃O₃S:** 60.36 %C, 3.65 %H, 11.46 %N.
**Measured:** 59.76 %C, 3.98 %H, 11.78 %N.
Melting point: **172-178 °C**

## Claims

1. The compounds of the formula I 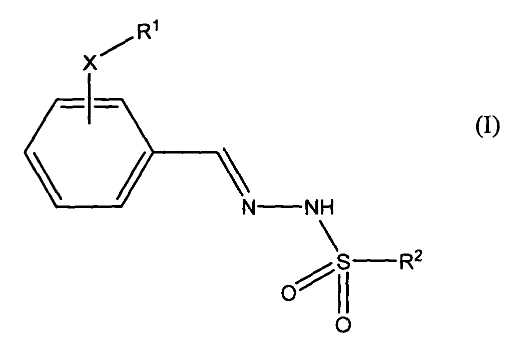 wherein
R¹ is a residue, which may be nonsubstituted, mono-, di-, or polysubstituted phenyl, benzyl, phenethyl or polycyclic aromatic ring or combination of five and sixmembered ring with one or more heteroatoms. Some examples are presented in the formula below 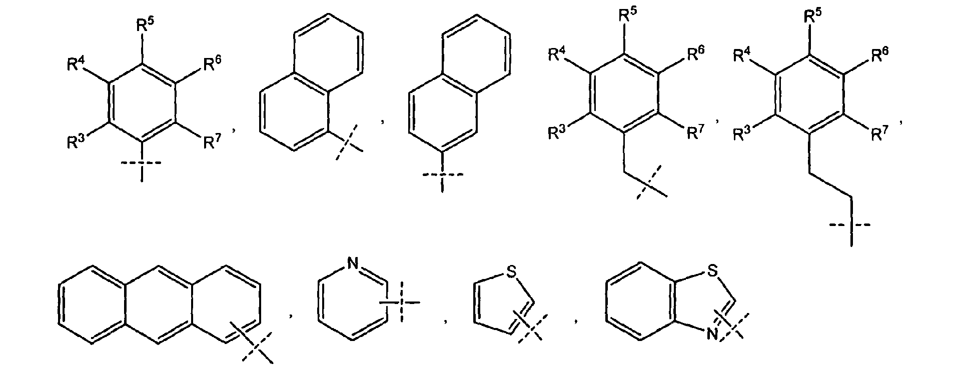 wherein R³, R⁴, R⁵, R⁶ in R⁷ = H, alkyl (C₁-C₆), OH, NH₂, SH, NO₂, SO₂NH₂, SOCH₃, SO₂CH₃, CF₃, F, Cl, Br, I, NHCOCH₃;
R³, R⁴, R⁵, R⁶ and R⁶ may form additional carbocyclic or heterocyclic ring with 1 , 2 or more heteroatoms,
R² represents nonsubstituted, mono, di, polysubstituted phenyl, benzyl or polycyclic aromatic ring of the formula 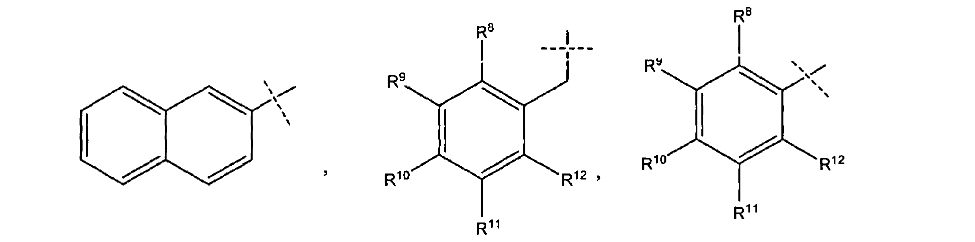 wherein R⁸, R⁹, R¹⁰, R¹¹ in R¹² = H, alkyl (C₁-C₆), OH, NH₂, SH, NO₂, SO₂NH₂, SOCH₃, SO₂CH₃, CF₃, F, Cl, Br, I, NHCOCH₃;
R⁸, R⁹, R¹⁰, R¹¹ and R¹² may form additional carbocyclic or heterocyclic ring with 1 , 2 or more heteroatoms,
X represents a residue of the formula: -OCH₂-, -NHCO-, -CONH, -OSO₂-, -COO-, -OOC-,-O-, -S-, -NH-
and pharmaceutically acceptable salts thereof;

2. A process for the preparation of the compounds having the formula I of Claim 1
**characterised in that**:
a) with the proviso that X = -OCH₂-
suitably substituted hydroxybenzaldehyde of formula (II) 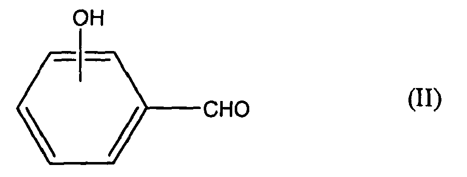 is converted in the presence of KF and suitable base with substituted arylalkylchloride of formula (III) 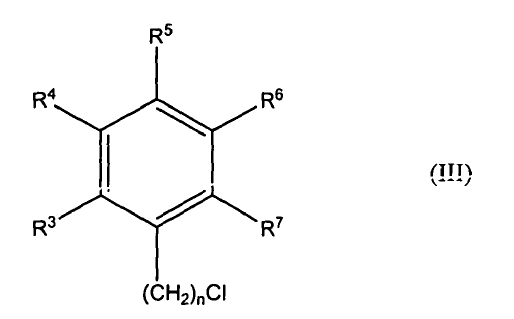 wherein n=1-3, R³, R⁴, R⁵, R⁶ and R⁷ have the same meanings as in the formula (I), or with substituted arylalkylchloride of formula (IV) 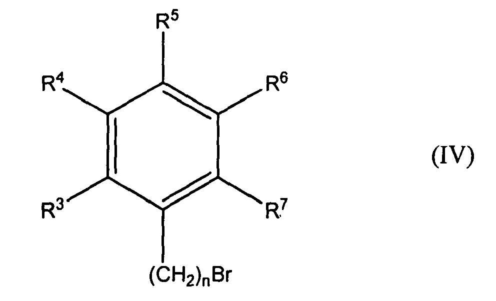 wherein n=1-3; R³, R⁴, R⁵, R⁶ and R⁷ have the same meanings as in the formula (I), to the compound (V) 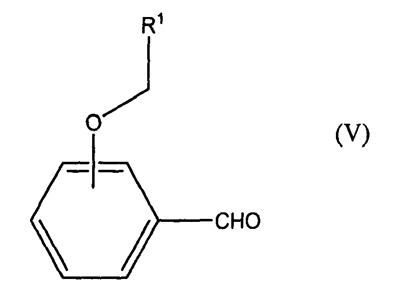 wherein R¹ has the same meaning as in the formula (I), which reacts with aryl-, arylalkyl-, or heteroaryl- sulfonohydrazide of the formula (VI) 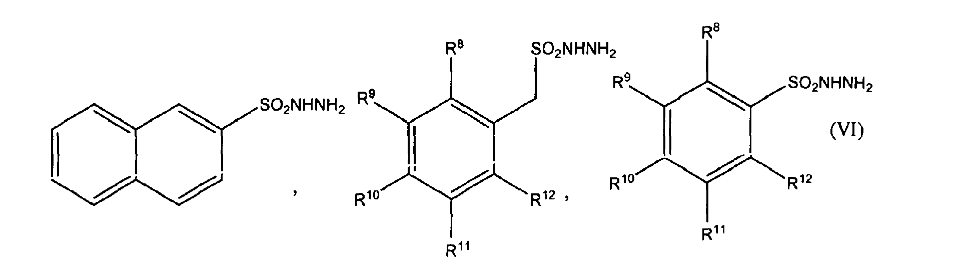 wherein R⁸, R⁹, R¹⁰, R¹¹ in R¹² have the same meanings as in the formula (I), to the compound of the formula (VII), 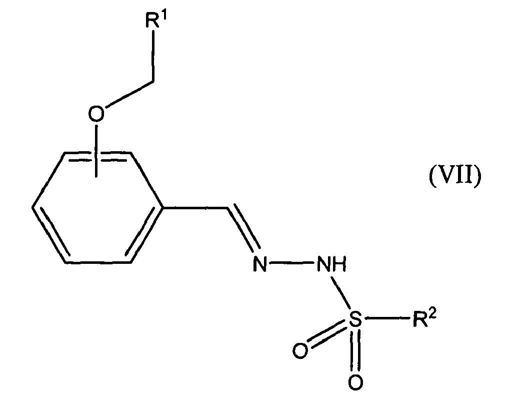 wherein R¹ in R² have the same meanings as in the formula (I);
b) with the proviso that X = -OSO₂-
suitably substituted hydroxybenzaldehyde of the formula (II) 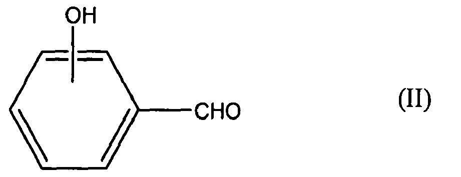 is converted with substituted arylsulfonylchloride or arylalkylsulfonylchloride of formula (VIII) 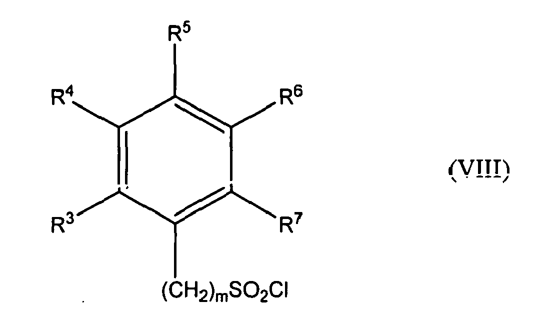 wherein m=0-2; R³, R⁴, R⁵, R⁶ and R⁷ have the same meanings as in the formula (I), to the compound of the formula (IX) 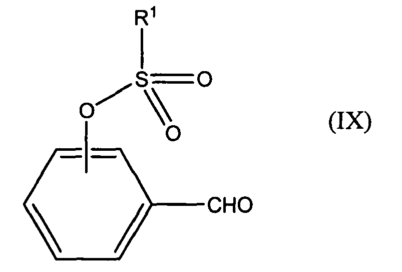 wherein R¹ has the same meaning as in the formula (I),
which is transformed with aryl-, arylalkyl-, or heteroaryl- sulfonohydrazide of the formula (VI) 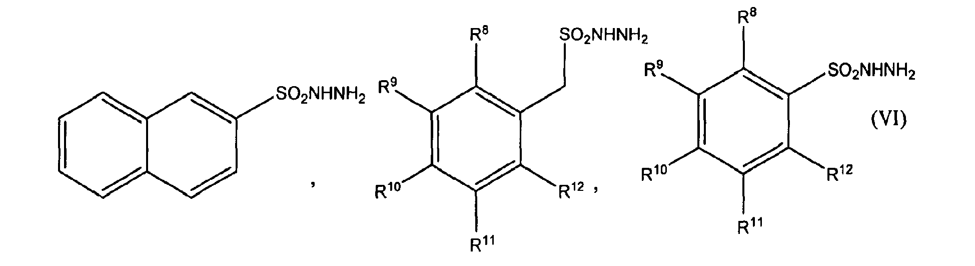 wherein R⁸, R⁹, R¹⁰, R¹¹ and R¹² have the same meanings as in the formula (I), to the compound of the formula (X), 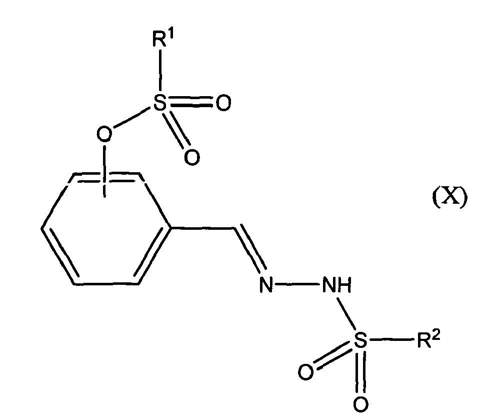 wherein R¹and R² have the same meanings as in the formula (I);
c) with the proviso that X = -NHCO-
suitably substituted nitrobenzaldehyde of the formula (XI) 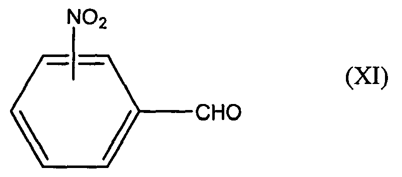 is protected with ethyleneglycol, to the compound of the formula (XII), 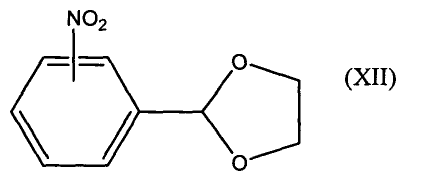 which by subjecting to reduction by catalytic hydrogenation, using Pd as a catalyst, and by the use of substituted benzoylchloride or arylalkanoylchloride of formula (XIII) 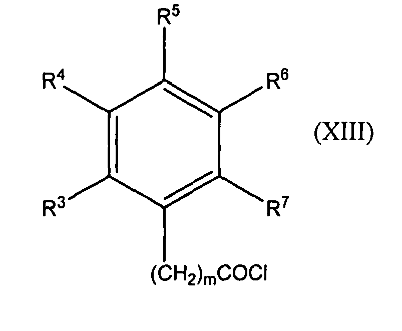 wherein m=0-2; R³, R⁴, R⁵, R⁶ and R⁷ have the same meanings as in the formula (I), is converted to the compound of the formula (XIV), 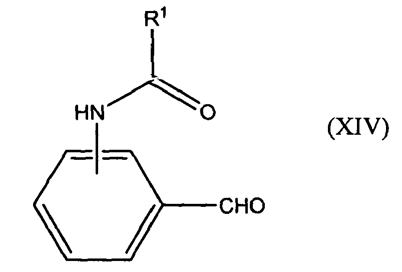 wherein R¹ has the same meaning as in the formula (I),
which is transformed with aryl-, arylalkyl-, or heteroaryl- sulfonohydrazide of the formula (VI) 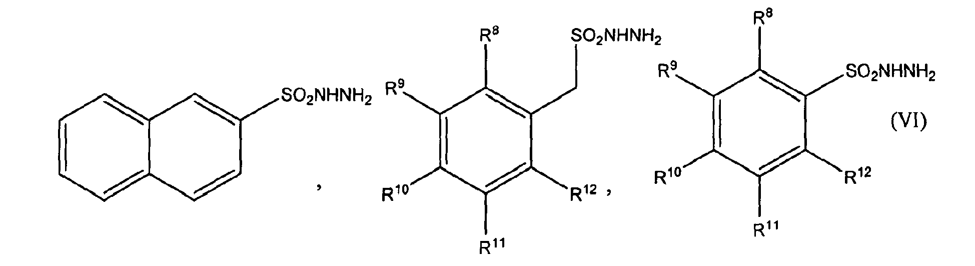 wherein R⁸, R⁹, R¹⁰, R¹¹ and R¹² have the same meanings as in the formula (I), to the compound of the formula (XV), 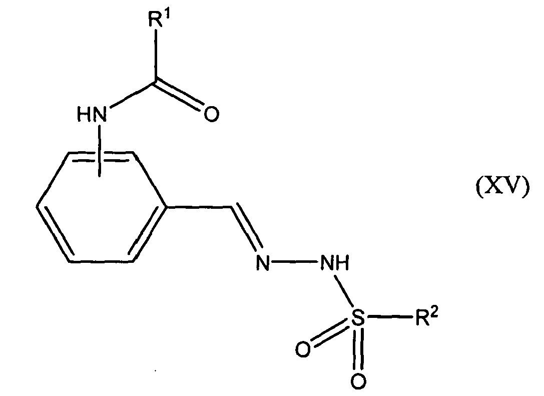 wherein R¹ and R² have the same meanings as in the formula (I);
d) with the proviso that X = -CONH-
suitably substituted carboxybenzaldehyde of the formula (XVI) 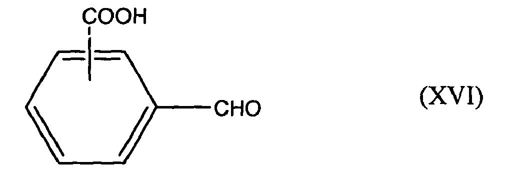 is reacted with aryl-, arylalkyl-, or heteroaryl- sulfonohydrazide of the formula (VI) 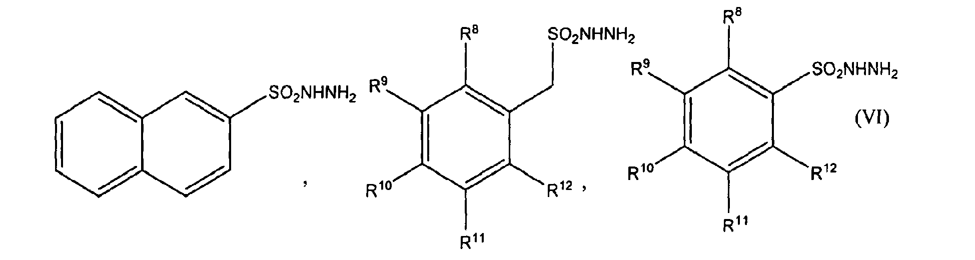 wherein R⁸, R⁹, R¹⁰, R¹¹ and R¹² have the same meanings as in the formula (I), to the compound of the formula (XVII), 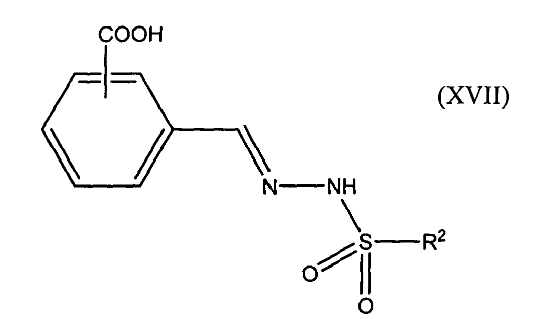 wherein R² has the same meaning as in the formula (I),
which is converted with aniline, arylamine or heteroarylamine derivative of the formula (XVIII) 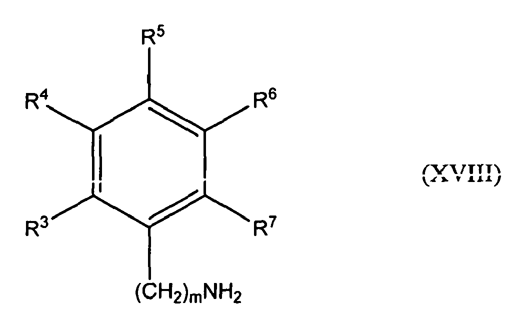 wherein m =0-2; R³, R⁴, R⁵, R⁶ and R⁷ have the same meanings as in the formula (I),
in the presence of EDC and HOBT or DCC or DPPA or BOP to the compound of the formula (XIX) 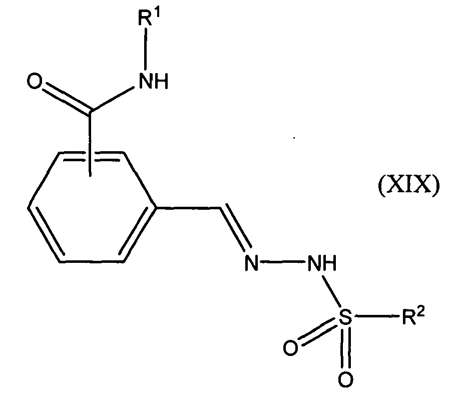 wherein R¹ and R² have the same meanings as in the formula (I);
e) with the proviso that X = -COO-
suitably substituted carboxybenzaldehyde of the formula (XVI) 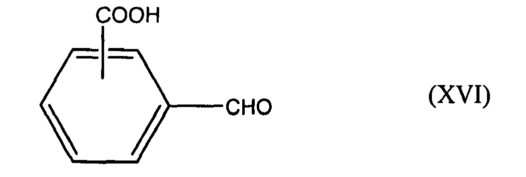 is reacted with aryl-, arylalkyl-, or heteroaryl- sulfonohydrazide of the formula (VI) 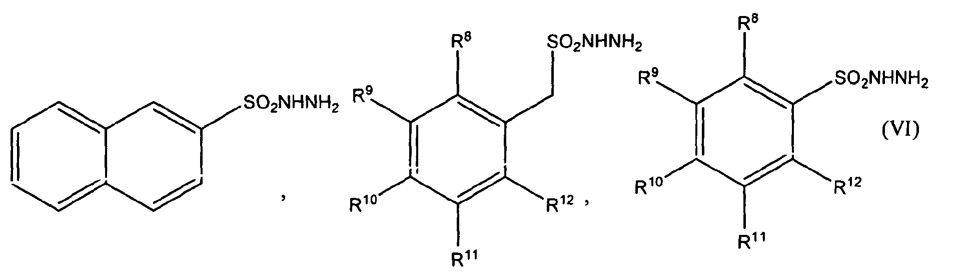 wherein R⁸, R⁹, R¹⁰, R¹¹ and R¹² have the same meanings as in the formula (I), to the compound of the formula (XVII), 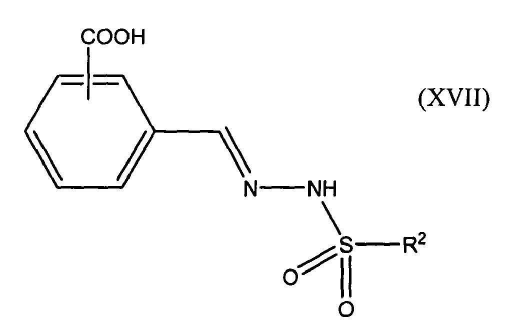 wherein R² has the same meaning as in the formula (I), which is converted with suitably substituted phenol or arylalkylalcohol of the formula (XX) 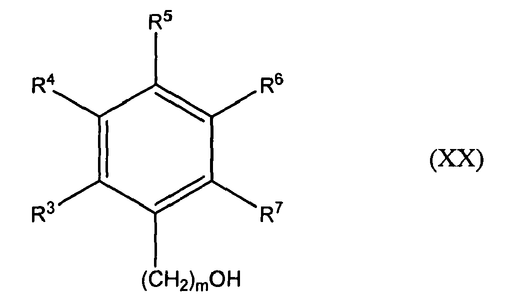 wherein m =0-2; R³, R⁴, R⁵, R⁶ and R⁷ have the same meanings as in the formula (I), in the presence of EDC and HOBT or DCC or DPPA or BOP to the compound of the formula (XXI) 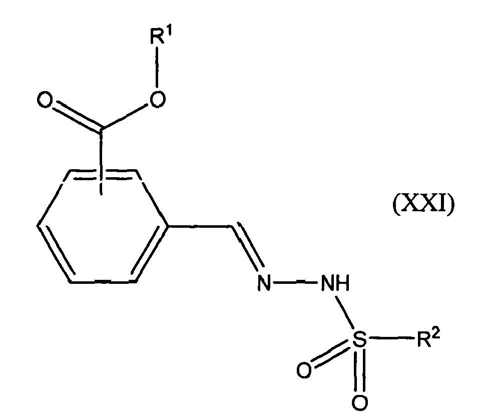 wherein R¹ and R² have the same meanings as in the formula (I);
f) with the proviso that X = -OOC-
suitably substituted hydroxybenzaldehyde of formula (II) 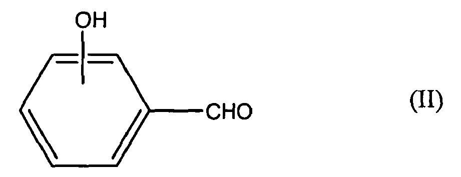 is transformed in the presence of EDC and HOBT or DCC or DPPA or BOP with substituted benzoic acid, or arylalcanoic acid of the formula (XXII) 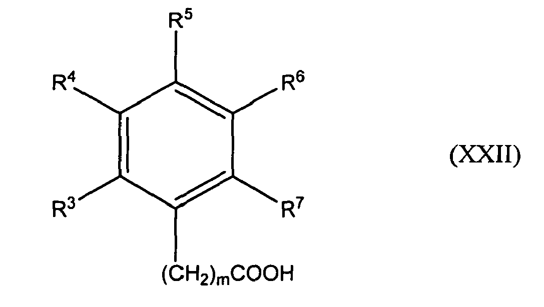 wherein m =0-2; R³, R⁴, R⁵, R⁶ and R⁷ have the same meanings as in the formula (I), or with substituted benzoylchloride or arylalkanoylchloride of formula (XIII) 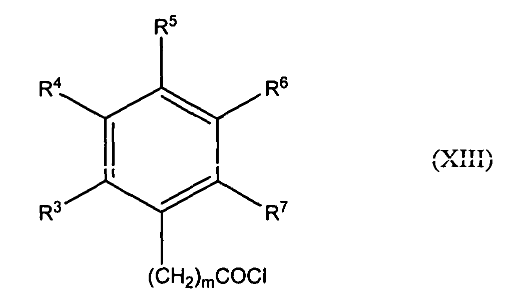 wherein m =0-2; R³, R⁴, R⁵, R⁶ and R⁷ have the same meanings as in the formula (I), to the compound of the formula (XXIII) 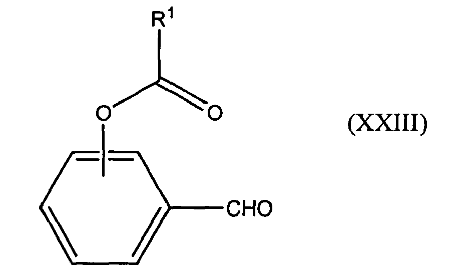 wherein R¹ has the same meaning as in the formula (I),
which is reacted with aryl-, arylalkyl-, or heteroaryl- sulfonohydrazide of the formula (VI) 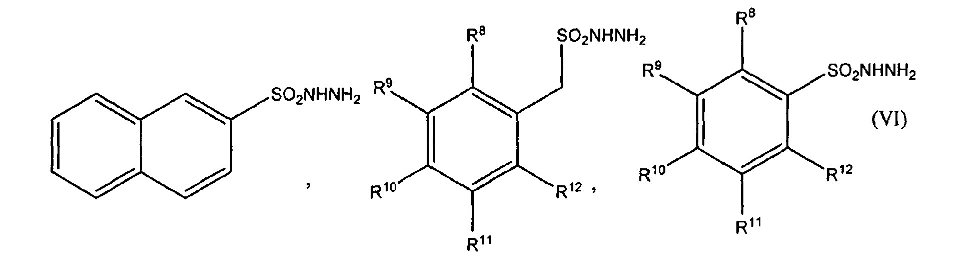 wherein R⁸, R⁹, R¹⁰, R¹¹ and R¹² have the same meanings as in the formula (I), to the compound of the formula (XXIV), 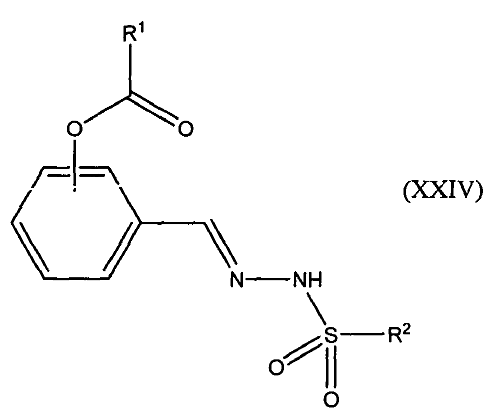 wherein R¹ and R² have the same meanings as in the formula (I);
g) with the proviso that X = -O-
suitably substituted aryloxybenzaldehyde or heteroaryloxybenzaldehyde of the formula (XXV), 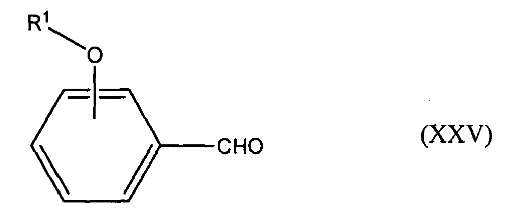
wherein R¹ has the same meaning as in the formula (I), is reacted with aryl-, arylalkyl-, or heteroaryl- sulfonohydrazide of the formula (VI) 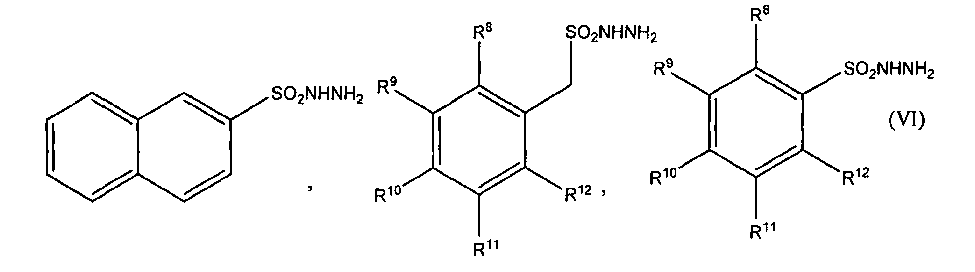 wherein R⁸, R⁹, R¹⁰, R¹¹ and R¹² have the same meanings as in the formula (I), to the compound of the formula (XXVI), 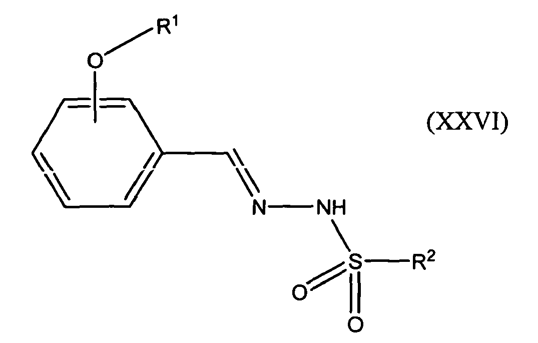 wherein R¹ and R² have the same meanings as in the formula (I);

3. the compounds having the formula I of Claim 1 for use as therapeutically active substances;

4. pharmaceutical compositions **characterised in that** they are comprising a therapeutically effective amount of the compound having the formula I of Claim 1 and pharmaceutically acceptable auxiliary substances.

5. the pharmaceutical compositions of Claim 4 **characterised in that** they are used as inhibitors of MurC and/or MurD.

6. the pharmaceutical compositions of Claim 4 **characterised in that** they are used against microbial infections in man or other mammals.
